# EUROPÄISCHE PATENTANMELDUNG

(11) **EP 1 704 885 A1**
(43) Veröffentlichungstag der Anmeldung: **27.09.2006**
(21) Anmeldenummer: 05006544.0
(22) Anmeldetag: 24.03.2005
(51) Int. Cl.: A61M 5/142, A61M 5/145

(54) **Vorrichtung für die dosierte Verabreichung eines fluiden Produkts**

(71) Anmelder: Disetronic Licensing AG, 3401 Burgdorf (CH)
(72) Erfinder: Peter, Daniel, 3172 Niederwangen (CH); Spoerri, Beat, 4578 Bibern (CH)
(74) Vertreter: Wess, Wolfgang

(57) **Zusammenfassung**

Vorrichtung für die dosierte Verabreichung eines fluiden Produkts, die Vorrichtung umfassend:
a) ein Behältnis (12) für das Produkt,
b) eine Fördereinrichtung (10, 13, 20), die auf das in dem Behältnis (12) befindliche Produkt wirkt und mittels einer axialen Abtriebsbewegung in eine Förderrichtung (V) Produkt aus dem Behältnis (12) fördert,
c) und eine in sich axial steife Gehäusestützstruktur (2), die das Behältnis (12) in und gegen die Förderrichtung (V) abstützt,
e) wobei die Gehäusestützstruktur (2) über die axiale Länge des Behältnisses (12) eine axiale Wärmeausdehnung aufweist, die in einem Einsatztemperaturbereich der Vorrichtung zumindest im Wesentlichen der axialen Wärmeausdehnung des Behältnisses (12) entspricht.

## Beschreibung

Die Erfindung betrifft Vorrichtungen für die dosierte Verabreichung von fluiden Produkten in biotechnologischen Anwendungen, vorzugsweise in medizinischen einschließlich tiermedizinischen und pharmazeutischen Anwendungen. Insbesondere betrifft sie Infusionsgeräte und Injektionsgeräte.

In Therapien kommt der Genauigkeit der Dosierung der verabreichten Produkte eine große Bedeutung zu, beispielsweise der Verabreichung von Insulin in der Diabetestherapie. Gebräuchlich sind Infusionsgeräte und Injektionsgeräte, die ein zu verabreichendes Produkt mittels einer motorisch betriebenen Hubkolbenpumpe im Falle von Infusionsgeräten oder mittels einer manuell betätigten Hubkolbenpumpe im Falle von Injektionsgeräten aus einem Produktreservoir ausschütten. Bei Infusionsgeräten wird der Hubkolben üblicherweise von einem Rotationsantrieb angetrieben, wobei die Rotationsbewegung des Antriebs mittels eines Spindeltriebs in die Linearbewegung des Kolbens übertragen wird. Bei Injektionsgeräten dient ein Spindeltrieb häufig der Auswahl der zu verabreichenden Produktdosis, während die Linearbewegung des Kolbens unmittelbar manuell bewirkt wird. Bei Injektionsgeräten sind ferner Zahnstangengetriebe gebräuchlich. Den genannten Beispielen von Verabreichungsgeräten ist gemein, dass die Dosiergenauigkeit maßgeblich davon abhängt, wie genau die Weglänge vorgegeben werden kann, die der Kolben für die Förderung einer bestimmten Produktdosis zurücklegen muss.

Infusionsgeräte und Injektionsgeräte der vorstehend beschriebenen Art werden beispielsweise durch die DE 198 40 992 A, die DE 198 22 031 C und die DE 199 00 827 C beschrieben.

Besondere Anforderungen an die Dosiergenauigkeit und -feinheit stellen Infusionsgeräte, mit denen das Produkt oft über längere Zeiträume in kleinen, diskreten Boli abgegeben wird. Dabei kann es vorkommen, dass konstruktive Merkmale, die im Grunde der Verbesserung der Dosiergenauigkeit dienen, gleichzeitig auch einen störenden Einfluss ausüben können, so beispielsweise die Fähigkeit der Okklusionserkennung. Ein Infusionsgerät mit einer vorteilhaft gestalteten, automatischen Okklusionserkennung wird in der DE 198 40 992 beschrieben, die diesbezüglich hiermit auch für die Zwecke der Erfindung in Bezug genommen wird. Ein weiteres Gerät mit Okklusionserkennung beschreibt die WO 01/72357 A2. Für die Okklusionserkennung wird die Fördereinrichtung im Ganzen über einen Sensor an dem Gehäuse des Infusionsgeräts abgestützt. Um zu verhindern, dass durch diese Art der Abstützung Relativbewegungen zwischen der Fördereinrichtung und dem Produktbehältrlls ermöglicht werden, schlägt die WO 01/72357 A2 für den Zusammenbau des Geräts vor, die gesamte Fördereinrichtung in Förderrichtung des Kolbens zunächst bis gegen einen von dem Gehäuse gebildeten Anschlag zu drücken, anschließend die Fördereinrichtung im Wesentlichen von dem Druck zu entlasten und schließlich eine Abschlusskappe in eine rückwärtige Gehäuseöffnung einzusetzen und mit dem Gehäuse zu verkleben. Die Kappe soll die Fördereinrichtung auf Anschlag gegen das Gehäuse halten. Als alternative Ausführung wird ferner vorgeschlagen, die Fördereinrichtung an ihrer von dem Kolben abgewandten Seite mittels eines elastischen Dichtrings an dem rückwärtigen Boden des Gehäuses abzustützen und einen zwischen der Rückseite der Fördereinrichtung und dem Gehäuseboden verbleibenden Hohlraum mit einem Füllmaterial auszugießen, beispielsweise mit Silikon. Das Füllmaterial soll im Wesentlichen inkompressibel sein, um den Sensor nicht zu entlasten.

Es ist eine Aufgabe der Erfindung, mit Vorrichtungen für die dosierte Verabreichung fluider Produkte die gewünschte Produktdosis genauer als bisher zu fördern.

Eine Vorrichtung für die dosierte Verabreichung eines fluiden Produkts, wie die Erfindung sie betrifft, umfasst ein Gehäuse, ein von dem Gehäuse aufgenommenes Behältnis für das Produkt und eine Fördereinrichtung, die auf das in dem Behältnis befindliche Produkt wirkt, um es aus dem Behältnis zu fördern. Das Behältnis wird von dem Gehäuse in einer definierten Ausrichtung gehalten. Vorzugsweise bildet eine Ampulle, beispielsweise eine Glasampulle, das Behältnis. Das Behältnis kann, wie bereits heute bei Ampullen üblich, fertig konfektioniert sein, indem es mit einer definierten Produktmenge gefüllt ist und auch bereits ein das Behältnis rückwärtig abdichtender Kolben in dem Behältnis aufgenommen ist. Fertig konfektionierte Ampullen dieser Art sind für die Selbstverabreichung von Insulin in der Diabetestherapie üblich. Bei dem Produkt kann es sich um das bereits genannte Insulin, ein Wachstumshormon und grundsätzlich jedes andere medizinisch oder beispielsweise kosmetisch wirksame Produkt handeln. Die erfindungsgemäße Vorrichtung ist bevorzugt für die Selbstverabreichung konzipiert.

Die Fördereinrichtung umfasst wenigstens ein Antriebsglied und wenigstens ein Abtriebsglied. Das Antriebsglied wird vorzugsweise motorisch, falls die Vorrichtung ein Infusionsgerät ist, und vorzugsweise manuell, falls die Vorrichtung ein Injektionsgerät ist, angetrieben, so dass es eine Antriebsbewegung ausführt. Das Antriebsglied und das Abtriebsglied sind miteinander mechanisch so gekoppelt, dass die Antriebsbewegung des Antriebsglieds eine Abtriebsbewegung des Abtriebsglieds bewirkt. Die Abtriebsbewegung ist oder umfasst eine Axialbewegung, die vorzugsweise über das Antriebsglied axial von dem Gehäuse abgestützt wird. Der Axialbewegung kann eine andere Bewegung oder können mehrere andere Bewegungen überlagert sein. Vorzugsweise ist die Abtriebsbewegung jedoch eine reine, lineare Axialbewegung.

Das Gehäuse kann mehrteilig, insbesondere zweiteilig, gebildet sein mit einer ersten Gehäusestruktur, die vorzugsweise eine Gehäusehüllstruktur, besonders bevorzugt eine zumindest im Wesentlichen geschlossene, äußere Hülle des Gehäuses bildet. Die erste Gehäusestruktur kann insbesondere aus Kunststoff geformt sein, beispielsweise im Spritzguss, d. h. sowohl dem Material nach als auch dem formgebenden Verfahren nach in herkömmlicher Weise. Das mehrteilige Gehäuse umfasst ferner eine zweite Gehäusestruktur, an der das Behältnis und vorzugsweise auch die Fördereinrichtung oder zumindest solche Komponenten der Fördereinrichtung axial abgestützt sind, deren axiale Wärmeausdehnung die Dosiergenauigkeit maßgeblich beeinflusst.

Die zweite Gehäusestruktur wird ihrer Funktion wegen nachfolgend als Gehäusestützstruktur oder einfach nur als Stützstruktur bezeichnet. Obgleich das Gehäuse bevorzugt über die Stützstruktur hinaus eine Hüllstruktur umfasst, kann die Stützstruktur in alternativen Ausführungen des Gehäuses dieses auch bereits allein bilden.

Sowohl in der Ausbildung des Gehäuses mit Hüllstruktur als auch in der Ausbildung ohne Hüllstruktur ist die Stützstruktur axial in sich steif. Dies kann insbesondere durch Einstückigkeit der Stützstruktur über die abzustützende axiale Länge des Behältnisses erzielt werden. Falls die Stützstruktur, wie bevorzugt, weitere Komponenten abstützt, kann die axiale Steifigkeit durch Einstückigkeit über eine entsprechend größere axiale Länge erzielt werden. Die Stützstruktur kann aber selbst aus mehreren, je in sich axial steifen Stützstrukturteilen gebildet sein, die axial steif miteinander verbunden sind, so dass die aus den mehreren Teilen erhaltene Stützstruktur insgesamt zumindest über die axiale Länge zwischen ihren wenigstens zwei Stützstellen wieder in sich axial steif ist.

Nach der Erfindung stützt die Stützstruktur das Behältnis in und gegen die Förderrichtung ab. Die Stützstruktur weist über die Länge des Behältnisses eine axiale Wärmeausdehnung auf, die in einem Einsatztemperaturbereich der Vorrichtung zumindest im Wesentlichen der axialen Wärmeausdehnung des Behältnisses entspricht. Falls das Behältnis wie bevorzugt direkt, d.h. ohne Zwischenkomponenten, in und gegen die Förderrichtung an der Stützstruktur abgestützt ist, werden die zu vergleichenden axialen Wärmeausdehnungen gemessen für den Axialabschnitt der Stützstruktur, der das Behältnis in und gegen die Förderrichtung abstützt, und für den Axialabschnitt des Behältnisses, der sich zwischen den Axialstützstellen des Behältnisses erstreckt. Für den Vergleich werden die axialen Längen bei einer Temperatur aus dem Einsatztemperaturbereich der Vorrichtung gemessen. Der Einsatztemperaturbereich überdeckt Temperaturen von -20°C bis +40°C.

Herkömmliche Verabreichungsvorrichtungen stützen das üblicherweise aus Glas gebildete Behältnis in und gegen die Förderrichtung an einem Kunststoffgehäuse ab. Die Wärmeausdehnungskoeffizienten der üblichen Kunststoffmaterialien unterscheiden sich von den Wärmeausdehnungskoeffizienten der üblichen Glasmaterialien um etwa den Faktor 10, d. h. um eine ganze Größenordnung. Entsprechend unterscheiden sich auch die axialen Wärmeausdehnungen der Gehäuse und Produktbehältnisse der herkömmlichen Verabreichungsvorrichtungen. Vor diesem Hintergrund wird im Sinne der Erfindung eine in [m] gemessene, axiale Wärmeausdehnung der Stützstruktur auch dann noch als im Wesentlichen gleich mit der axialen Wärmeausdehnung des Behältnisses angesehen, wenn die beiden unter der Erfindung in Rede stehenden axialen Wärmeausdehnungen sich um nicht mehr als 500 % unterscheiden. Bevorzugter unterscheiden sich die axialen Wärmeausdehnungen um nicht mehr als 300 %. Die Stützstruktur sollte aus einem Material gefertigt sein, das einen axialen Wärmeausdehnungskoeffizienten aufweist, der sich um nicht mehr als 500 % von dem Wärmeausdehnungskoeffizienten des Materials des Behältnisses unterscheidet. Falls die Stützstruktur nicht homogen aus einem Material hergestellt ist, für das ein einziger Wärmeausdehnungkoeffizient gilt, so sollten die vorstehenden Aussagen für sämtliche in der Stützstruktur verarbeitete Materialien gelten.

Das Behältnis kann insbesondere aus Glas gefertigt sein, wie dies heute üblich ist.

Die Stützstruktur ist idealerweise aus dem gleichen Material wie das Behältnis gefertigt, was bei Verwendung eines Glasbehältnisses jedoch kaum möglich sein wird. Eine metallische Stützstruktur, die die erfindungsgemäßen Bedingungen in Bezug auf die axiale Wärmeausdehnung erfüllt, ist ein weiterer bevorzugter Kandidat. Eine weitere Option ist es, die Stützstruktur als Verbundkörper zu bilden, beispielsweise mit einer Stützmatrix, welche die erfindungsgemäßen Bedingungen bezüglich ihrer axialen Wärmeausdehnungen erfüllt, und einem Kunststoffmaterial, mit dem die Stützmatrix umgossen und in dem die Stützmatrix verankert ist. Ein geeigneter Verbundkörper kann auch als faserverstärkter Kunststoffkörper gebildet sein, in dessen Kunststoffmatrix axial sich erstreckende Fasern, beispielsweise Metallfasern, eingebettet sind.

Falls das Gehäuse mehrteilig mittels Hüllstruktur und Stützstruktur gebildet wird, ist die Stützstruktur mit der Hüllstruktur vorzugsweise formschlüssig oder form- und kraftschlüssig verbunden und kann insbesondere in die Hüllstruktur eingesetzt sein. Falls die Hüllstruktur ein Gussteil ist, kann die Stützstruktur beispielsweise auch mit dem Material der Hüllstruktur umgossen sein. Ein Stoffschluss zwischen der Hüllstruktur und der Stützstruktur ist ebenfalls möglich.

Nicht nur, aber insbesondere, falls die Stützstruktur zur Erzielung des erfindungsgemäßen Wärmeausdehnungsverhaltens als Verbundkörper gebildet ist, kann auf eine zusätzliche Gehäusestruktur wie beispielsweise die Hüllstruktur, in die die Stützstruktur eingesetzt ist, verzichtet werden. Hüll- und Stützstruktur fallen in solch einem Fall zusammen. Hierbei muss die kombinierte Hüll- und Stützstruktur das erfindungsgemäße Wärmeausdehnungsverhalten nicht überall gleichmäßig aufweisen. Es genügt ein axial abschnittsweise erfindungsgemäßes Wärmeausdehnungsverhalten, beispielsweise durch Einbettung von Fasermaterial nur in einem maßgeblichen Axialabschnitt.

Ein Vorteil der erfindungsgemäßen Stützstruktur ist, dass das Behältnis axial nicht mittels einer Ausgleichsfeder an dem Gehäuse abgestützt werden muss, sondern mittels axial beidseitigen Anschlägen axial fixiert werden kann. Bei einem Ausgleich der axialen Längenvariationen mittels Ausgleichsfeder ändert sich die axiale Position des Behältnisses in Bezug auf die Fördereinrichtung. Die Positionsänderung ist jedoch der Dosiergenauigkeit der Förderung abträglich. Für die axiale Abstützung in und gegen die Förderrichtung ist das Behältnis deshalb vorzugsweise sowohl in als auch gegen die Förderrichtung auf Anschlag gegen die Stützstruktur, so dass es relativ zu der Stützstruktur axial fixiert ist. Falls das Behältnis trotz der erfindungsgemäßen Stützstruktur mittels einer Ausgleichsfeder an der Stützstruktur abgestützt wird, so sind zumindest die Variationen der axialen Längen von Behältnis und Stützstruktur deutlich kleiner als die Variationen bei den herkömmlichen Verabreichungsvorrichtungen, so dass auch in diesem Fall noch die Dosiergenauigkeit der Förderung gegenüber den herkömmlichen Verabreichungsvorrichtungen verbessert wird, wenn eine Ausgleichsfeder zum Einsatz gelangt. Die ausgleichenden Federwege sind dann kürzer. Eine axial steife Abstützung zwischen Anschlägen wird allerdings bevorzugt. Für die Abstützung kann die Stützstruktur eine Stützschulter unmittelbar für das Behältnis sowohl in die Förderrichtung als auch gegen die Förderrichtung bilden, was besonders bevorzugt wird. In oder gegen die Förderrichtung oder grundsätzlich auch in beide Richtung kann die Abstützung aber auch indirekt, d.h. erst über eine oder mehrere weitere Komponenten, beispielsweise die Fördereinrichtung, realisiert sein, wobei die betreffende weitere Komponente, beispielsweise die Fördereinrichtung, oder die gegebenenfalls mehreren Komponenten letztlich wie vorstehend beschrieben für die Schaffung einer spielarmen Abstützung des Behältnisses an der Stützstruktur axial abgestützt sind. Bei solch einer indirekten Abstützung wird in Axialrichtung eine ebenfalls steife Abstützung der betreffenden Komponenten untereinander, d.h. Stoß an Stoß, bevorzugt. Allerdings gelten auch hier die vorstehenden Ausführungen bezüglich eines eventuell zwischen zwei Komponenten angeordneten Federelements.

Von den für die axiale Abstützung, vorzugsweise Fixierung, erforderlichen Stützschultern wird eine von der Stützstruktur vorzugsweise unmittelbar, d. h. in einem Stück, gebildet. Eine dieser Stützschulter axial gegenüberliegende Stützgegenschulter wird vorzugsweise von einem Stützelement gebildet, das mit der Stützstruktur lösbar verbunden ist und bevorzugt ein Abschlusselement eines Aufnahmefachs für das Behältnis bildet. Die Stützstruktur und das lösbare Stützelement weisen zusammenwirkende Eingriffseinrichtungen auf, um die lösbare, aber im verbundenen Zustand feste Verbindung miteinander bilden zu können. Bevorzugt handelt es sich bei den beiden Eingriffseinrichtungen um Gewinde, so dass das lösbare Stützelement in das Aufnahmefach eingeschraubt oder auf das Aufnahmefach aufgeschraubt werden kann. Das lösbare Stützelement bildet vorzugsweise die in Förderrichtung vordere der Stützschultern. Obgleich weniger bevorzugt, soll jedoch nicht ausgeschlossen sein, dass das Behältnis von hinten in das Aufnahmefach eingesetzt wird, so dass in diesem Fall das vordere Stützelement bevorzugt in einem Stück von der Stützstruktur gebildet oder unlösbar mit der Stützstruktur verbunden sein kann, während das hintere Stützelement in diesem Fall vorzugsweise lösbar und im verbundenen Zustand fest mit der Stützstruktur verbunden ist. Das Aufnahmefach kann auch so gebildet sein, dass das Behältnis von der Seite eingesetzt wird. Die Stützschulter und die Stützgegenschulter können hierfür in einem geringen Ausmaß elastisch nachgiebig sein, um das Behältnis beispielsweise einclippen zu können. Die Stützstruktur weist die erfindungsgemäße Eigenschaft der am Behältnis orientierten Wärmeausdehnung zumindest in ihrem Axialabschnitt zwischen der Stützschulter und der Stützgegenschulter für das Behältnis auf.

In einer bevorzugten Weiterbildung stützt die Stützstruktur auch die Fördereinrichtung oder wenigstens eine Komponente der Fördereinrichtung axial ab. Die Stützstruktur weist in einem Axialabschnitt, der sich von dem Ort der Abstützung der Fördereinrichtung oder der wenigstens einen Komponente bis zu einem in Förderrichtung vorderen Ende der Fördereinrichtung oder der Komponente erstreckt, eine axiale Wärmeausdehnung auf, die sich bevorzugterweise um höchstens 500 % und noch bevorzugter um höchstens 200 % von der axialen Wärmeausdehnung der Fördereinrichtung oder der wenigstens einen Komponente unterscheidet, wobei die axiale Wärmeausdehnung der Fördereinrichtung über ihren vorstehend genannten Axialabschnitt gemessen wird.

Die Fördereinrichtung kann an der Stützstruktur in und gegen die Förderrichtung auf Anschlag sein und somit von der Stützstruktur axial fixiert werden, wie dies bezüglich des Behältnisses beschrieben wurde. In einer anderen Ausführung ist die Fördereinrichtung an der Stützstruktur axial nur in eine Richtung per Anschlag und in die andere über eine Federeinrichtung abgestützt. Die Federeinrichtung kann vorteilhaft dazu dienen, ein Axialspiel eines Rotationsglieds der Fördereinrichtung zu eliminieren oder wenigstens zu reduzieren.

Ist die Verabreichungsvorrichtung mit einem Kraftsensor ausgestattet, um eine Okklusion und/oder ein Leck in einem das Produkt führenden Teil der Vorrichtung feststellen zu können, so kann der Kraftsensor eine Federeinrichtung im genannten Sinne bilden. Um eine Okklusions- und/oder eine Lecksituation detektieren zu können, ist die Fördereinrichtung gegen die Förderrichtung über den Kraftsensor an der Stützstruktur abgestützt. Die Fördereinrichtung ist als solche mit der Stützstruktur nicht fest verbunden, sondern drückt zumindest bei der Produktförderung oder dem Versuch der Produktförderung an einer Kontaktfläche, die vorzugsweise punktförmig ist, gegen die Stützstruktur oder ein axial steif mit der Stützstruktur verbundenes Kontaktelement. Der Kraftsensor kann axial steif sein, beispielsweise in der Ausbildung als Piezoelement, bevorzugter ist der Kraftsensor jedoch axial elastisch nachgiebig. In solch einer bevorzugten Ausbildung ist die Fördereinrichtung gegen eine elastische Rückstellkraft des Kraftsensors relativ zu der Stützstruktur gegen die Förderrichtung bewegbar und somit über den Kraftsensor an der Stützstruktur axial schwimmend gelagert. Der Kraftsensor kann insbesondere ein elastisches Biegeelement sein, das mit Dehnungsmessstreifen versehen ist, um über die mit der elastischen Durchbiegung verbundene Dehnung die von der Fördereinrichtung auf die Stützstruktur wirkende axiale Reaktionskraft aufzunehmen. Alternativ könnte ein steifes Sensorelement axial federnd abgestützt sein und beispielsweise die axiale Auslenkung als Weg gemessen werden. Als Kraftsensor wird im Sinne der Erfindung jeder Sensor verstanden, aus dessen Messgröße auf die Kraft oder den Druck geschlossen werden kann, mit der die Fördereinrichtung auf das Produkt im Behältnis wirkt.

Die Kopplung zwischen dem Antriebsglied und dem Abtriebsglied ist vorzugsweise ein Flankeneingriff, zu dessen Bildung das Antriebsglied und das Abtriebsglied je wenigstens eine Eingriffsflanke aufweisen. Vorzugsweise sind sowohl die wenigstens eine Eingriffsflanke des Antriebsglieds unmittelbar an dem Antriebsglied als auch die wenigstens eine Eingriffsflanke des Abtriebsglieds unmittelbar an dem Abtriebsglied geformt. Die Antriebsbewegung kann eine Axialbewegung sein, wie dies insbesondere der Fall sein kann, wenn die Vorrichtung ein Injektionsgerät ist. Bevorzugter ist die Antriebsbewegung jedoch eine Rotationsbewegung und in diesem Fall besonders bevorzugt um eine Achse, entlang der das Abtriebsglied die Abtriebsbewegung ausführt.

Das Abtriebsglied kann bei der Abtriebsbewegung unmittelbar auf das in dem Reservoir befindliche Produkt wirken, indem es beispielsweise selbst einen Hubkolben bildet oder mit einem Hubkolben permanent verbunden ist. Es kann aber auch nur lose gegen einen Hubkolben drücken. Möglich ist auch eine Ausführung, in der das Abtriebsglied erst über ein Übertragungsglied oder mehrere Übertragungsglieder auf ein unmittelbar auf das Produkt wirkendes Förderelement, beispielsweise einen Hubkolben, wirkt, wenn es die Abtriebsbewegung ausführt. So kann die Fördereinrichtung insbesondere teleskopierbar sein, wie dies in der DE 197 17 107 A beschrieben ist, die diesbezüglich in Bezug genommen wird. Je zwei benachbarte, im Flankeneingriff befindliche Teleskopstufen bilden in solch einer Ausbildung ein Antriebsglied und ein Abtriebsglied gemäß der Erfindung.

In bevorzugter Ausführung weist die Stützstruktur in einem Axialabschnitt, der sich von dem Flankeneingriff bis zu einem in Förderrichtung vorderen Ende des Abtriebsglieds erstreckt, eine axiale Wärmeausdehnung auf, die sich von der axialen Wärmeausdehnung des Abtriebsglieds um höchstens 500 % und vorzugsweise höchstens 200% unterscheidet. Die Erstreckung des Axialabschnitts der Stützstruktur ist hierbei auf eine in Förderrichtung vorderste Position des Abtriebsglieds bezogen.

Flankeneingriffe, wie sie insbesondere von Gewindeeingriffen und Zahneingriffen bekannt sind, weisen fertigungsbedingt quer zu den Eingriffsflanken ein axiales Spiel auf, das der Dosiergenauigkeit abträglich sein kann, beispielsweise wenn bei einer Ausschüttung ein Syphoning-Effekt auftritt, d.h. eine Saugsituation im Behältnis.

In noch einer Weiterbildung ist jedoch eine zweite Spielreduziereinrichtung vorgesehen, die sowohl mit dem Antriebsglied als auch mit dem Abtriebsglied je in einem Einstelleingriff ist, in dem die Spielreduziereinrichtung relativ zu dem Abtriebsglied und dem Antriebsglied in solche eine Einstellposition verstellt und in der Einstellposition so gesichert ist, dass das dem Flankeneingriff inhärente Axialspiel gegenüber den bekannten, auf Flankeneingriff beruhenden Kopplungen reduziert oder vorzugsweise gänzlich eliminiert ist. Der Einstelleingriff mit einem der beiden Glieder aus Antriebsglied und Abtriebsglied entspricht dem Flankeneingriff zwischen dem Antriebsglied und dem Abtriebsglied. Der andere Einstelleingriff gibt die Verstellbewegung der Spielreduziereinrichtung entlang ihres Verstellwegs vor. Die in diesem Einstelleingriff verfügbare Verstellweglänge ist vorzugsweise so lang, dass die Spielreduziereinrichtung in ihrer Einstellposition nicht auf Anschlag mit dem betreffenden Glied ist, sondern noch innerhalb der in diesem Eingriff verfügbaren Verstellweglänge. Die beiden Einstelleingriffe können insbesondere auch von gleicher Art oder vollkommen gleich sein. Im letzteren Fall geben sie den Verlauf des Verstellwegs gemeinsam vor.

Der Einstelleingriff mit dem Antriebsglied ist vorzugsweise form- und kraftschlüssig, besonders bevorzugt ist der Einstelleingriff ein Gewindeeingriff. Bezüglich des Einstelleingriffs mit dem Abtriebsglied gilt vorzugsweise das Gleiche. Die Ausbildung beider Einstelleingriffe der zweiten Spielreduziereinrichtung als Gewindeeingriff bietet sich insbesondere dann an, wenn der Flankeneingriff zwischen dem Antriebsglied und dem Abtriebsglied ebenfalls eine Gewindeeingriff ist, wie die Erfindung es bevorzugt. Es ist jedoch beispielsweise auch möglich, den Einstelleingriff, der die Verstellbewegung vorgibt, als Eingriff eines Eingriffsglieds der Spielreduziereinrichtung in eine Führungsbahn rein formschlüssig zu gestalten und mittels einer Elastizitätskraft den anderen der beiden Einstelleingriffe form- und kraftschlüssig zu bilden. Der die Verstellbewegung vorgebende Einstelleingriff ist in bevorzugten Ausführungen kontinuierlich in dem Sinne, dass das Axialspiel zwischen dem Antriebsglied und dem Abtriebsglied von seiner fertigungsbedingten Ausgangsgröße bis vorzugsweise auf den Wert Null kontinuierlich im Einstelleingriff verringert werden kann, wie dies beispielsweise der bevorzugte Gewindeeingriff ermöglicht. Der Gewindeeingriff bietet ferner den Vorteil, dass die Spielreduziereinrichtung durch den Einstelleingriff selbst in jeder über den Verstellweg eingenommenen Position axial gestützt wird.

In bevorzugten Ausführungen ist die zweite Spielreduziereinrichtung an einem der Glieder aus Antriebsglied und Abtriebsglied gegen Axialbewegungen relativ zu dem betreffenden Glied gesichert. Die Sicherung kann insbesondere so gestaltet sein, dass die Spielreduziereinrichtung die Antriebsbewegung mitmacht, wenn die Sicherung zwischen dem Antriebsglied und der Spielreduziereinrichtung besteht, und die Abtriebsbewegung mitmacht, wenn die Sicherung zwischen der Spielreduziereinrichtung und dem Abtriebsglied besteht.

Im beispielhaften Fall des Gewindeeingriffs kann die Sicherung der zweiten Spielreduziereinrichtung in dem Einstelleingriff in der Einstellposition allein durch Selbsthemmung bewirkt werden. Vorzugsweise ist die Spielreduziereinrichtung in ihrer Einstellposition in dem gesicherten Einstelleingriff jedoch stoffschlüssig gesichert. Dies gilt auch, falls es sich hierbei um einen Gewindeeingriff handelt. Die stoffschlüssige Sicherung erfolgt vorzugsweise in dem Einstelleingriff mit dem Antriebsglied. Eine Sicherung an dem Abtriebsglied wäre grundsätzlich jedoch in kinematischer Umkehr ebenfalls möglich. Anstatt die Sicherung allein in einem einzigen der beiden Einstelleingriffe zu realisieren, kann die Sicherung auch erst im Zusammenwirken der Spielreduziereinrichtung mit dem Antriebsglied und dem Abtriebsglied realisiert werden, in diesem Fall vorzugsweise dadurch, dass die Spielreduziereinrichtung sowohl an dem Antriebsglied als auch an dem Abtriebsglied elastisch abgestützt ist.

Es soll nicht ausgeschlossen werden, dass eines aus Antriebsglied und Abtriebsglied eine Zahnstange und das andere ein in die Zahnstange eingreifender Mitnehmer ist. Solche Zahnstangentriebe sind bekannt, beispielsweise von Injektionspens, so dass auf Details nicht eingegangen werden muss. Für solch einen Zahnstangentrieb kann die zweite Spielreduziereinrichtung mittels eines weiteren Mitnehmers gebildet werden, so dass zwei Mitnehmer in die gleiche Zahnreihe eingreifen. Durch Verstellung des axialen Abstands der beiden Mitnehmer kann auch bei einem derartigen Zahnstangentrieb das toleranzbedingt von Hause aus vorhandene Axialspiel reduziert werden.

Falls die Fördereinrichtung teleskopierbar ist, so wird vorteilhafterweise zwischen jedem Paar von im Eingriff zwischen dem Antriebsglied und dem Abtriebsglied, vorzugsweise Flankeneingriff, befindlichen Teleskopstufen eine erfindungsgemäße, zweite Spielreduziereinrichtung vorgesehen. Im Falle einer teleskopierbaren Fördereinrichtung erstreckt sich ein die erfindungsgemäße Wärmeausdehnung mit Bezug auf die Fördereinrichtung aufweisender Axialabschnitt der Stützstruktur vorteilhafterweise von dem vorderen Ende eines vordersten Abtriebsglieds bis zu dem Eingriff des hintersten Abtriebsglieds mit einem Antriebsglied der Fördereinrichtung.

In bevorzugten Verabreichungsvorrichtungen ist das Antriebsglied ein Rotationsglied, das um eine Rotationsachse rotatorisch bewegbar gelagert ist. Das Abtriebsglied ist ein translatorisch in eine Translationsrichtung bewegbares Translationsglied. Eine rotatorische Antriebsbewegung des Antriebsglieds in eine Antriebsrichtung bewirkt die axiale Abtriebsbewegung des Abtriebsglieds in die Förderrichtung. Handelt es sich bei der Verabreichungsvorrichtung um ein Infusionsgerät, so ist das rotatorisch bewegbare Antriebsglied vorzugsweise so abgestützt, dass es sich relativ zu einem Lagerkörper in und gegen die Translationsrichtung des Abtriebsglieds nicht bewegen kann. Da das Abtriebsglied sich gegen die Förderrichtung vorzugsweise an dem Antriebsglied abstützt, würde eine unerwünschte Axialbewegung des Antriebsglieds bereits aufgrund des bei den bekannten Drehlagerungen unvermeidbaren Axialspiels seiner für die Rotationsbewegung erforderlichen Drehlagerung in Reaktion auf die Abtriebsbewegung des Abtriebsglieds stattfinden.

In noch einer bevorzugten Weiterbildung ist für derartige Fördereinrichtungen deshalb eine dritte Spielreduziereinrichtung vorgesehen, um das der Drehlagerung des Antriebsglieds inhärente Axialspiel zu reduzieren und vorzugsweise zu eliminieren. Die Drehlagerung umfasst den Lagerkörper, der das Antriebsglied um seine Rotationsachse drehbar lagert. Der Lagerkörper kann mehrteilig sein, wobei die mehreren Teile axial steif miteinander verbunden sind, vorzugsweise ist er jedoch einteilig. Er kann ferner unmittelbar von dem Gehäuse der Vorrichtung gebildet werden. Bevorzugt wird es jedoch, wenn der Lagerkörper separat gebildet und in das Gehäuse eingesetzt ist. Ferner entspricht es bevorzugten Ausführungsformen, wenn der Lagerkörper eine Lagerhülse ist, die das Antriebsglied zumindest in einem axialen Abschnitt umgibt.

Um das Axialspiel der Drehlagerung zu reduzieren, sind wenigstens zwei axiale Stützflächen der Drehlagerung axial steif mit dem Lagerkörper und wenigstens zwei weitere axiale Stützflächen der Drehlagerung axial steif mit dem Antriebsglied verbunden. In axial steifer Verbindung können die Stützflächen entweder unmittelbar von dem Lagerkörper oder dem Antriebsglied gebildet werden, oder es wird die betreffende Stützfläche von einem separaten Körper gebildet, der dann jedoch axial steif, vorzugsweise vollkommen steif, mit entweder dem Lagerkörper oder dem Antriebsglied verbunden ist. Vorzugsweise wird wenigstens eine der Stützflächen von der dritten Spielreduziereinrichtung gebildet, die entweder mit dem Antriebsglied oder vorzugsweise dem Lagerkörper in einem Einstelleingriff in solch eine Einstellposition verstellt und in der Einstellposition an dem Lagerkörper oder dem Antriebsglied axial so gesichert ist, dass das Axialspiel der Drehlagerung reduziert und vorzugsweise eliminiert wird. Falls das Antriebsglied und das Abtriebsglied Stufen einer teleskopierbaren Fördereinrichtung sind, wie die DE 197 17 107 A sie beispielsweise beschreibt, so bildet die Drehlagerung die Drehlagerung der Eingangsstufe der Fördereinrichtung.

Die erste und/oder zweite und/oder dritte Spielreduziereinrichtung ist oder sind vorzugsweise eingliedrig als ein einziges Einstellglied gebildet, das in beiden Einstelleingriffen und entsprechend dem zu reduzierenden Axialspiel zumindest axial steif ist. Im Falle einer mehrgliedrigen Spielreduziereinrichtung sollte solch eine Spielreduziereinrichtung zumindest dann axial in sich steif sein, wenn sie in der Einstellposition gesichert ist. So könnte beispielsweise eine zweigliedrige Spielreduziereinrichtung ein erstes Einstellglied aufweisen, das in einem Einstelleingriff mit dem Antriebsglied ist, und ein zweites Einstellglied, das in einem Einstelleingriff mit dem Abtriebsglied ist. Die beiden Einstellglieder würden bis in die Einstellposition hinein axial relativ zueinander verstellt und müssten in der Einstellposition axial aneinander festgelegt werden oder sich selbsttätig aneinander festlegen, um die axiale Steifigkeit zu erhalten.

Die Erfindung wirkt vorteilhaft mit jeder der Spielreduziereinrichtungen und jeder möglichen Kombination von zweien der Spielreduziereinrichtungen und besonders vorteilhaft mit allen dreien zusammen.

Die die vorstehend lediglich im Zusammenhang mit der Vermeidung von Axialspiel aufgrund von Wärmeausdehnungen beschriebene Stützstruktur hat auch ohne die erfindungsgemäßen Wärmeausdehnungseigenschaften Vorteile. Die Stützstruktur kann ungeachtet dieser Eigenschaft als Stützstruktur oder Lagerstruktur für das Behältnis und die Fördereinrichtung dienen, so dass bei einem aus der Stützstruktur und einer Hüllstruktur gebildeten Gehäuse die Hüllstruktur von der Funktion der axialen Abstützung oder gänzlich von der Funktion der Lagerung des Behältnisses und der Fördereinrichtung und vorzugsweise von sämtlichen Stütz- und sonstigen Lagerfunktionen befreit ist. So kann die Stützstruktur insbesondere so ausgelegt sein, dass das Behältnis, die Fördereinrichtung und vorzugsweise weitere Komponenten der Vorrichtung, beispielsweise die gesamte Elektronik, von der Stützstruktur axial abgestützt und vorzugsweise in jeder Hinsicht gelagert werden. Die an der Stützstruktur abgestützten und vorzugsweise gänzlich von ihr gelagerten Komponenten können vorteilhafterweise bei der Montage in einem ersten Schritt mit der Stützstruktur verbunden werden, bevor anschließend in einem weiteren Schritt die Hüllstruktur in Form von beispielsweise zwei Schalen um die Stützstruktur montiert wird. Die Montage vereinfacht sich hierdurch erheblich. Insbesondere muss die Hüllstruktur nicht mehr wie bislang üblich tragend konzipiert sein, sondern übernimmt vorzugsweise nur noch eine Schutzfunktion für die umhüllten Komponenten der Vorrichtung. Ferner kann die Hüllstruktur frei von Stütz- und vorzugsweise frei von jeglicher Lagerfunktion im Hinblick auf Design, insbesondere Handhabungskomfort, gebildet sein. Die Anmelderin behält es sich vor, auf eine Vorrichtung mit solch einem Gehäuse aus Stützstruktur und Hüllstruktur einen separaten Schutz zu richten, insbesondere auf eine Vorrichtung für die dosierte Verabreichung eines fluiden Produkts, die wenigstens die folgenden Komponenten umfasst: ein Behältnis für das Produkt; eine Fördereinrichtung, die auf das in dem Behältnis befindliche Produkt wirkt und mittels einer axialen Abtriebsbewegung in eine Förderrichtung Produkt aus dem Behältnis fördert; eine axial in sich steife Gehäusestützstruktur, die das Behältnis und die Fördereinrichtung axial abstützt, indem sie das Behältnis mit einer vorderen Stützstelle in die Förderrichtung und die Fördereinrichtung mit einer Stützstelle in oder/und gegen die Förderrichtung axial abstützt; und eine Gehäusehüllstruktur, die zumindest einen größeren Teil der Gehäusestützstruktur umhüllt. Die Vorrichtung kann vorteilhafterweise im Sinne der Erfindung weitergebildet sein, so dass bevorzugte Ausführungen solch einer Vorrichtung wie vorstehend zur Erfindung beschrieben ausgebildet sind. Insbesondere kann sie entsprechend dem Merkmal d) von Anspruch 1, einem der Unteransprüche und jeder Kombination der Unteransprüche ausgebildet sein. Unter dem Aspekt der Trennung von axialer Stütz- und vorzugsweise jeglicher Lagerfunktion einerseits und Schutzfunktion und/oder Designfunktion andererseits können die in der vorliegenden Anmeldung in Bezug auf das Behältnis beschriebenen Eigenschaften der Wärmeausdehnung der Stützstruktur stattdessen auch auf die Fördereinrichtung oder nur axial bewegte Komponenten der Fördereinrichtung bezogen sein, während die in Bezug auf das Behältnis beschriebenen Eigenschaften der Wärmeausdehnung der Stützstruktur zwar vorteilhafterweise in solchen Ausführungen ergänzend ebenfalls gelten, aber nicht gelten müssen.

Bevorzugte Ausgestaltungen der Erfindung werden auch in den Unteransprüchen und durch die Kombinationen der Unteransprüche beschrieben.

Ausführungsbeispiele der Erfindung werden nachfolgend anhand von Figuren erläutert. An den Ausführungsbeispielen offenbar werdende Merkmale bilden je einzeln und in jeder Merkmalskombination die Gegenstände der Ansprüche und die vorstehend beschriebenen Ausgestaltungen vorteilhaft weiter. Es zeigen:
- Figur 1: eine Verabreichungsvorrichtung eines ersten Ausführungsbeispiels in einem Längsschnitt,
- Figur 2: ein Teil der Verabreichungsvorrichtung in einem anderen Längsschnitt,
- Figur 3: ein vergrößertes Detail der Figur 2,
- Figur 4: eine Spielreduziereinrichtung in einer alternativen Ausführung,
- Figur 5: ein Einstellglied der Spielreduziereinrichtung der Figur 4,
- Figur 6: eine Verabreichungsvorrichtung eines zweiten Ausführungsbeispiels in einem Längsschnitt,
- Figur 7: ein Detail X der Figur 6,
- Figur 8: einen Federring einer Spielreduziereinrichtung des zweiten Ausführungsbeispiels,
- Figur 9: Komponenten der Verabreichungsvorrichtung des zweiten Ausführungsbeispiels in einer Explosionsdarstellung und
- Figur 10: das Translationsglied des zweiten Ausführungsbeispiels.

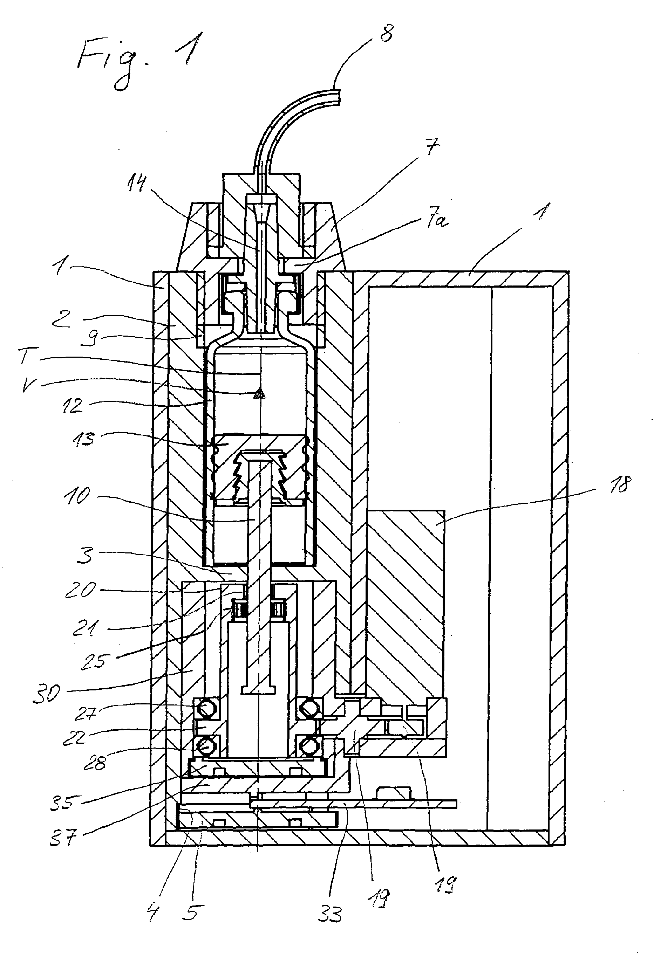

Figur 1 zeigt in einem Längsschnitt als Beispiel einer Verabreichungsvorrichtung ein Infusionsgerät. Das Gerät weist ein Gehäuse mit einer ersten Gehäusestruktur 1, und einer zweiten Gehäusestruktur 2, ein mit einem injizierbaren Produkt gefülltes Behältnis 12 und eine Fördereinrichtung auf, die das Produkt dosiert aus dem Behältnis 12 und durch einen sich anschließenden Katheter 8 fördert, um es zu verabreichen. Die Verabreichung kann insbesondere subkutan vorgenommen werden, wie dies beispielsweise in der Diabetestherapie üblich ist. Die erste Gehäusestruktur 1 umgibt die zweite Gehäusestruktur 2 und wird im folgenden als Hüllstruktur 1 bezeichnet. Die zweite Gehäusestruktur 2 stützt Komponenten des Infusionsgeräts und wird im folgenden als Stützstruktur 2 bezeichnet.

Das Behältnis 12 weist an einem vorderen Ende einen Auslass 14 auf, über den der Behältnisinnenraum mit dem Katheter 8 verbunden ist. In dem Behältnis 12 ist ein Kolben 13 entlang einer Translationsachse T in eine Förderrichtung V auf den Behältnisauslass 14 zu bewegbar aufgenommen. Das Behältnis 12 ist an seinem rückwärtigen Ende offen. Allerdings dichtet der Kolben 13 das Behältnis 12 zu dem rückwärtigen Ende hin ab.

Die Hüllstruktur 1 bildet eine feste Hülle, in der die Stützstruktur 2, die von einem Stützkörper 2 einstückig gebildet wird, eingesetzt und befestigt ist und ein Chassis des Gehäuses bildet. Die Hüllstruktur 1 und der Stützkörper 2 bilden das Gehäuse der Vorrichtung zumindest im Wesentlichen. Der Stützkörper 2 bildet einen ersten Aufnahmeraum, in dem das Behältnis 12 eingesetzt ist, und einen zweiten Aufnahmeraum für die Fördereinrichtung. Das Behältnis 12 ist mit seinem hinteren freien Rand auf Anschlag gegen einen, eine Stützschulter bildenden, nach radial einwärts ragenden Stützsteg 3 des Stützkörpers 2. Der von dem Stützkörper 2 gebildete erste Aufnahmeraum weist an dem vorderen Ende eine Öffnung auf, durch die das Behältnis 12 eingesetzt wird. Nach dem Einsetzen des Behältnisses 12 wird die Öffnung mit einem Deckel 7 verschlossen. Der Deckel 7 ist mit dem Stützkörper 2 verschraubt, der hierfür mit einem Gewinde 9 versehen ist. Der Stützkörper 2 und der Deckel 7 könnten auch mit je einer anderen Eingriffseinrichtung für den lösbaren Eingriff versehen sein, beispielsweise mit kooperierenden Rasteinrichtungen. Der Deckel 7 bildet ferner das Verbindungselement zwischen dem Katheter 8 und dem Auslass 14 des Behältnisses 12. Der Deckel 7 weist zu der Stützschulter des Stützstegs 3 eine Gegenstützschulter an einem Gegenstützsteg 7a auf, die gegen einen vorderen Rand des Behältnisses 12 und dadurch das Behältnis 12 auf Anschlag gegen den Stützsteg 3 drückt, so dass das Behältnis 12 relativ zu dem Stützkörper 2 axial festgelegt ist. Der Deckel 7 bildet somit ein vorderes Abschlusselement 7a, und der Stützsteg 3 bildet ein hinteres Abschlusselement 3 des ersten Aufnahmeraums. Der erste Aufnahmeraum ist ferner so geformt, dass das Behältnis 12 die korrekte Position und Ausrichtung in Bezug auf die Translationsachse T hat. Im Ergebnis stützt der Stützkörper 2 das Behältnis 12 axial in und gegen die Förderrichtung V formschlüssig, d.h. durch Kontakt mit den als Anschlägen dienenden Stegen 3 und 7a, ab. Die Fördereinrichtung wird in gleicher Weise axial zwischen anderen Anschlägen des Stützkörpers 2 abgestützt.

Die Fördereinrichtung umfasst den Kolben 13, ein Abtriebsglied 10, ein Antriebsglied 20 und einen motorischen Drehantrieb. Das Abtriebsglied 10 bildet ein Translationsglied der Fördereinrichtung, und das Antriebsglied 20 bildet ein Rotationsglied der Fördereinrichtung.

Das Abtriebsglied 10 ist eine Kolbengewindestange, d. h. eine Kolbenstange, die mit einem Gewinde versehen ist. Im Ausführungsbeispiel ist das Abtriebsglied 10 mit einem Außengewinde 11 versehen, das in dem Längsschnitt der Figur 2 erkennbar ist. Das Abtriebsglied 10 durchragt den Stützsteg 3, so dass es in den ersten Aufnahmeraum und den zweiten Aufnahmeraum des Stützkörpers 2 hinein ragt. An seinem vorderen Ende ist das Abtriebsglied 10 mit dem Kolben 13 verschraubt. Die Schraubverbindung wird mit dem Einsetzen des Behältnisses hergestellt. Der Stützsteg 3 führt das Abtriebsglied 10 linear entlang der Translationsachse T. Der Stützsteg 3 sichert das Abtriebsglied 10 so gegen eine Verdrehung relativ zu dem Stützkörper 2. Das Gewinde 11 wird im Ausführungsbeispiel hierfür von wenigstens einer axialen Nut oder Abplattung durchbrochen, in die der Stützsteg 3 eingreift.

Das Antriebsglied 20 ist gänzlich in dem zweiten Aufnahmeraum des Stützkörpers 2 angeordnet. Es ist rotationssymmetrisch zur Translationsachse T. Es ist hülsenförmig und kann daher auch als Antriebshülse bezeichnet werden. Das Antriebsglied 20 bildet an einem vorderen Hülsenende einen nach radial einwärts ragenden Steg, den das Abtriebsglied 10 durchragt. Das Antriebsglied 20 bildet an dem einwärts ragenden Steg ein Innengewinde 21, das mit dem Außengewinde 11 des Abtriebsglieds 10 in einem Gewindeeingriff ist.

Das Antriebsglied 20 ist um die Translationsachse T drehbar und relativ zu dem Stützkörper 2 axial nicht bewegbar gelagert. Es weist in einem hinteren Bereich einen nach radial auswärts ragenden, umlaufenden Steg 22 auf, der mit einer Außenverzahnung versehen ist. Über die Außenverzahnung wird das Antriebsglied 20 um die Translationsachse T drehangetrieben. Seinen Drehantrieb erhält es von einem Drehmotor 18, der in einem weiteren Aufnahmeraum aufgenommen ist. Der weitere Aufnahmeraum wird von der Hüllstruktur 1 gebildet und ist von den beiden Aufnahmeräumen des Stützkörpers 2 getrennt. Der Motor 18 treibt das Abtriebsglied 20 über ein Zahnradgetriebe mit Stirnzahnrädern 19 an, von denen ein Ausgangszahnrad 19 mit der Außenverzahnung des Abtriebsglieds 20 kämmt. Infolge des Gewindeeingriffs zwischen dem Antriebsglied 20 und dem Abtriebsglied 10 und der Linearführung des Abtriebsglieds 10, wird bei einem Drehantrieb des Antriebsglieds 20 durch dessen rotatorische Antriebsbewegung eine axiale Abtriebsbewegung des Abtriebsglieds 10 in die Förderrichtung V bewirkt. Das durch die Kolbenbewegung verdrängte Produkt wird über den Katheter 8 ausgeschüttet und auf diese Weise verabreicht.

Wie jeder Gewindeeingriff ist auch der Gewindeeingriff als solcher zwischen dem Abtriebsglied 10 und dem Antriebsglied 20 mit einem Axialspiel behaftet. Die Dosiergenauigkeit der Ausschüttung ist daher zumindest im Ausmaß dieses inhärenten Axialspiels mit einer Ungenauigkeit behaftet. Im Falle beispielsweise eines Ansaugens des Kolbens 13 aufgrund Syphoning oder bei mechanischen Stößen oder Druckdifferenzen zwischen dem Gehäuseinnenraum und der Umgebung kann es daher vorkommen, dass die Flanken des Außengewindes 11 des Abtriebsglieds 10 von den vortreibenden Gewindeflanken des Gewindes 21 abheben. Die axiale Position des Kolbens 13 ist daher im Ausmaß des Axialspiels der Gewinde 11 und 21 unbestimmt.

Allerdings ist eine Spielreduziereinrichtung vorgesehen, die mittels eines Einstellglieds 25 gebildet ist und die das Axialspiel zwischen dem Abtriebsglied 10 und dem Antriebsglied 20 nahezu eliminiert.

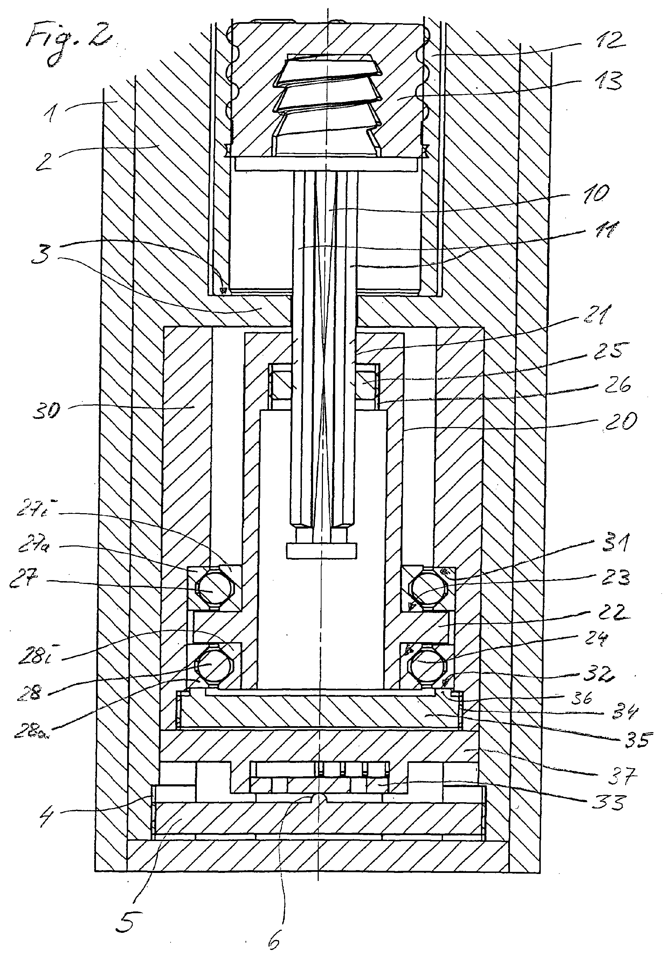

Aus den Figuren 2 und 3 sind Konstruktion und Wirkung der Spielreduziereinrichtung am besten ersichtlich. Die Spielreduziereinrichtung wird von einem einstückigen Einstellglied 25 gebildet. Das Einstellglied 25 ist in einem Einstelleingriff mit dem Abtriebsglied 10 und in einem weiteren Einstelleingriff mit dem Antriebsglied 20. Das Einstellglied 25 und die beiden Einstelleingriffe sind so gestaltet, dass das Axialspiel zwischen den Gewinden 11 und 21 deutlich reduziert oder vorzugsweise gänzlich aufgehoben wird.

Im Ausführungsbeispiel ist das Einstellglied 25 als Gewindemutter mit einem Innengewinde und einem Außengewinde gebildet. Mit seinem Innengewinde ist das Einstellglied 25 in einem Gewindeeingriff mit dem Außengewinde 11 des Abtriebsglieds 10. Mit seinem Außengewinde ist es in einem Gewindeeingriff mit einem Innengewinde 26 des Antriebsglieds 20. Das Innengewinde 26 ist dem Außengewinde 11 zugewandt und axial unmittelbar hinter dem Gewinde 21 gebildet. Das Innengewinde und das Außengewinde des Einstellglieds 25 liegen auf der gleichen axialen Höhe, so dass das Einstellglied 25 axial dünn sein kann und die Spielreduziereinrichtung dementsprechend axial, d. h. entlang der Translationsachse T, kurz baut. Das Innengewinde 26 muss lediglich so lang sein, dass ein sicherer Einstelleingriff mit dem Einstellglied 25 gewährleistet ist und das Einstellglied 25 darüber hinaus in diesem Einstelleingriff noch so weit verstellt werden kann, dass die angestrebte Reduzierung des Axialspiels der Gewinde 11 und 21 herbeigeführt werden kann. Das Innengewinde 26 weist eine Steigung auf, die es erlaubt, das Einstellglied 25 bei bestehendem Gewindeeingriff zwischen den Gewinden 11 und 21 und zwischen dem Gewinde 11 und dem Innengewinde des Einstellglieds 25 im Gewindeeingriff mit dem Innengewinde 26 zu verstellen.

Figur 3 zeigt in vergrößerter Darstellung den Gewindeeingriff der Gewinde 11 und 21 und den Einstelleingriff zwischen dem Gewinde 11 des Abtriebsglieds 10 und dem Innengewinde des Einstellglieds 25. Das Innengewinde des Einstellglieds 25 weist die gleiche Steigung wie das Außengewinde 11 auf. Vorzugsweise ist die Steigung des Außengewindes des Einstellglieds 25 und des Innengewindes 26 größer oder kleiner als die Steigung der Gewinde 11 und 21, allerdings so geringfügig, dass die Verstellung des Einstellglieds 25 im Einstelleingriff möglich ist.

Für die Axialspiekeduzierung ist das Einstellglied 25 in seinem Einstelleingriff mit dem Abtriebsglied 10 so eingestellt, dass seine bezüglich der Förderrichtung V rückwärtigen Gewindeflanken 25f in Kontakt mit den vorderen Gewindeflanken 11 f des Außengewindes 11 sind, während gleichzeitig die vorderen Flanken 21 f des antreibenden Gewindes 21 in Kontakt mit den rückwärtigen Flanken 11 f des Gewindes 11 des Abtriebsglieds 10 sind. Hierfür wird das Einstellglied 25 in seinem Einstelleingriff mit dem Antriebsglied 20 relativ zu dem Antriebsglied 20 gegen die Förderrichtung V verstellt, bis dieser Zustand des Flankenkontakts hergestellt ist. In diesem Zustand wird das Einstellglied 25 an dem Antriebsglied 20 fixiert und dadurch gesichert. Die Sicherung wird im Ausführungsbeispiel adhäsiv hergestellt, indem ein Adhäsionsmittel in den Einstelleingriff des Einstellglieds 25 mit dem Antriebsglied 20 eingebracht ist. Andere Möglichkeiten der stoffschlüssigen Verbindung zwischen dem Einstellglied 25 und dem Antriebsglied 20 sind jedoch ebenfalls denkbar, beispielsweise eine Laserverschweißung in der Einstellposition. Falls das Innengewinde 26, wie bevorzugt, eine andere Steigung als die Gewinde 11 und 21 hat, kann bereits hierdurch allein oder in Kombination mit einer stoffschlüssigen Verbindung die axiale Sicherung erzielt werden. Die Einstellposition sollte so gewählt werden, dass durch die Spielreduzierung keine oder jedenfalls keine praktisch relevanten Presskräfte auf das Abtriebsglied 10 ausgeübt werden. Die Einstellposition ist daher so gewählt, dass in dem Gewindeeingriff der Gewinde 11 und 21 ein sehr geringes Restspiel verbleibt, das jedoch deutlich kleiner als das diesem Eingriff allein, d. h. ohne Spielreduzierung, inhärente Gewindespiel ist.

Im Ausführungsbeispiel ist die Einstellposition des Einstellglieds 25 so gewählt, dass das Gewinde 21 das Antriebsgewinde des Abtriebsglieds 20 bleibt. Die Einstellposition könnte jedoch auch so gewählt sein, dass bei der Einstellung das Einstellglied 25 gegen die rückwärtigen Gewindeflanken des Gewindes 11 bewegt wird und in diesem Fall das Einstellglied 25 den Vortrieb des Abtriebsglieds 10 übernimmt. Bevorzugt wird jedoch die für das Ausführungsbeispiel gewählte und aus Figur 3 ersichtliche Einstellposition des Einstellglieds 25.

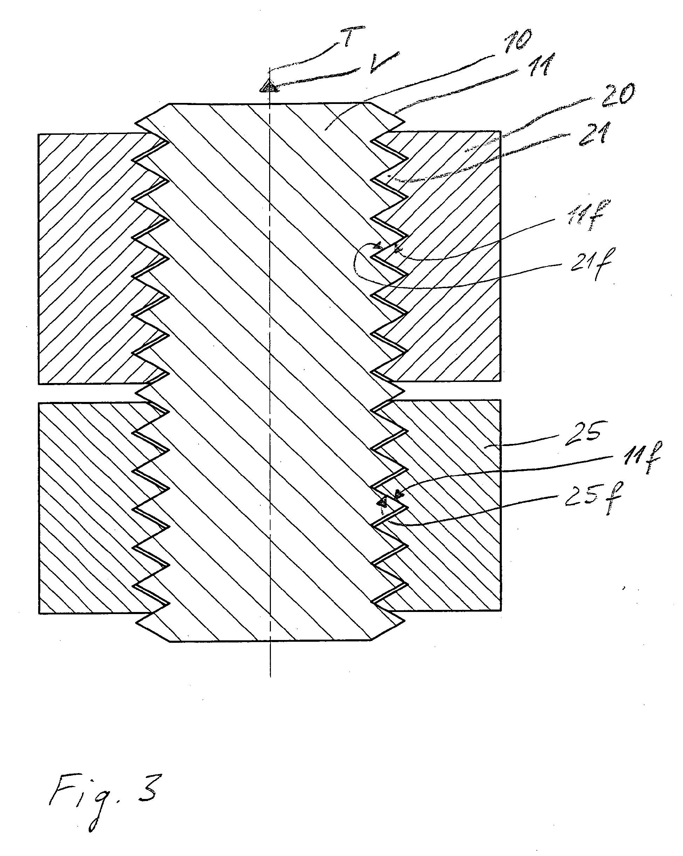

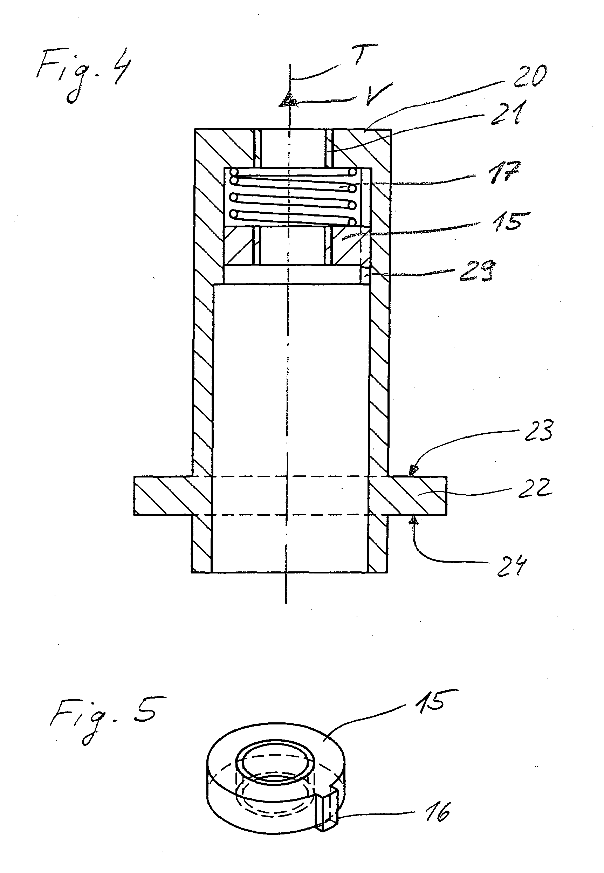

Ein alternatives Ausführungsbeispiel einer Spielreduziereinrichtung ist aus den Figuren 4 und 5 erkennbar. Im Vergleich zu den Komponenten des Ausführungsbeispiels der Figuren 1 bis 3 sind lediglich das Antriebsglied 20 und das im alternativen Ausführungsbeispiel mit "15" gekennzeichnete Einstellglied modifiziert, während die übrigen Komponenten, insbesondere das Abtriebsglied 10, unverändert sind. Als weiteren Unterschied umfasst die alternative Spielreduziereinrichtung zusätzlich ein elastisches Rückstellelement 17 in Form einer mechanischen Druckfeder.

Das Einstellglied 15 ist wie bereits das Einstellglied 25 in die das Abtriebsglied 20 bildende Hülse eingesetzt. Allerdings ist das Einstellglied 15 mit dem Abtriebsglied 20 axial linear verschiebbar und verdrehgesichert verbunden. Der Einstelleingriff des Einstellglieds 15 mit dem Antriebsglied 20 ist daher eine Linearführung. Die Linearführung wird von einer axialen, geraden Führungsbahn 29 an der Mantelinnenfläche des Abtriebsglieds und einem in die Führungsbahn 29 eingreifenden Eingriffsglied 16 (Fig. 5) des Einstellglieds 15 gebildet. Die Führungsbahn 29 wird in die Förderrichtung V von dem nach radial einwärts ragenden Steg des Antriebsglieds 20 begrenzt, der das Antriebsgewinde 21 bildet. Rückwärtig ist die Führungsbahn 29 offen, so dass das Einstellglied 15 eingeschoben werden kann. Zuvor wird noch das Rückstellelement 17 eingesetzt. Das Rückstellelement 17 ist in Förderrichtung an dem das Antriebsgewinde 21 bildenden Steg des Antriebsglieds 20 und gegen die Förderrichtung an dem Einstellglied 15 abgestützt. Figur 4 zeigt diesen Zustand vor dem Zusammenbau mit dem Abtriebsglied 10.

Für den Zusammenbau wird zuerst das Einstellglied 15 mit dem Rückstellelement 17 in das Antriebsglied 20 in den Einstellgriff mit der Führungsbahn 29 eingesetzt und mit einer gewissen Kraft gegen das Rückstellelement 17 gedrückt. Das Abtriebsglied 10 wird anschließend zuerst mit dem Einstellglied 15 und dann mit dem Antriebsgewinde 21 verschraubt. Im Einstelleingriff drücken die rückwärtigen Gewindeflanken des Innengewindes des Einstellglieds 15 mit einer Elastizitätskraft gegen die in die Förderrichtung V weisenden Gewindeflanken des Gewindes 11 des Abtriebsglieds 10. Im Ergebnis wird für den Gewindeeingriff der Gewinde 11 und 21 durch die zwei Einstelleingriffe des Einstellglieds 15 der gleiche Zustand erhalten, wie er in Figur 3 dargestellt ist. In der alternativen Spielreduziereinrichtung wird das Einstellglied 15 somit durch die Elastizitätskraft des Rückstellelements 17 in der Einstellposition gesichert.

Das Infusionsgerät des Ausführungsbeispiels weist als eine Besonderheit, wie sie jedoch grundsätzlich aus der DE 198 40 992 A bekannt ist, eine Okklusionserkennung auf, der ebenfalls ein Axialspiel inhärent ist, unter dem die Dosiergenauigkeit leiden kann. Dieses inhärente, zweite Axialspiel hat seine Ursache darin, dass die gesamte Fördereinrichtung, insbesondere das Abtriebsglied 10 und das Antriebsglied 20 über einen Sensor 33 an dem Stützkörper 2 axial abgestützt ist. Der Sensor 33 dient der Ermittlung der Kraft, die erforderlich ist, um den Kolben 13 entlang der Translationsachse T zu bewegen. Der Sensor kann beispielsweise auf einer Dehnungsmessung beruhen. Letztlich wird mittels des Sensors 33 eine den Fluiddruck in dem Behältnis 12 repräsentierende, physikalische Größe gemessen, um bei der Produktverabreichung eine etwaige Okklusion oder ein etwaiges Leck möglichst frühzeitig zu erkennen. Was die Okklusions- und/oder Leckerkennung und den Sensor 33 anbetrifft, wird im Folgenden lediglich auf die das Axialspiel betreffenden Aspekte eingegangen und im Übrigen auf die als Beispiel in Bezug genommene DE 198 40 992 A verwiesen.

Für die Okklusions- und/oder Leckerkennung ist die Fördereinrichtung, wie bereits erwähnt, über den Sensor 33 axial abgestützt. Dies bedeutet, dass das Antriebsglied 20, an dem sich das Abtriebsglied 10 im Antriebseingriff der Gewinde 11 und 21 axial abstützt, nicht axial steif mit dem Stützkörper 2 verbunden, sondern relativ zu dem Stützkörper 2 axial bewegbar gelagert ist, um nämlich den Fluiddruck in dem Behältnis 12, genauer gesagt den Differenzdruck zur Umgebung, ermitteln zu können. Um die axial bewegbare Lagerung zu erhalten, ist das Antriebsglied 20 in einem Lagerkörper 30 drehgelagert und an dem Lagerkörper 30 axial gesichert. Der Lagerkörper 30 ist in den zweiten Aufnahmeraum des Stützkörpers 2 eingesetzt und gegen Verdrehung gesichert. Der Stützkörper 2 führt den Lagerkörper 30 axial durch Gleitkontakt. Der Lagerkörper 30, und damit zusammen das Abtriebsglied 10 und das Antriebsglied 20, ist über den Sensor 33 an dem Stützkörper 2 axial so abgestützt, dass der Sensor 33 die gesamte, zwischen dem Lagerkörper 30 und dem Stützkörper 2 wirkende und gegen die Förderrichtung V gerichtete Axialkraft aufnimmt. In Förderrichtung V ist der Lagerkörper 30 gegen den Stützkörper 2 auf Anschlag. Der Lagerkörper 30 lagert auch den Motor 18 und das Getriebe 19 der Fördereinrichtung. Im Ausführungsbeispiel ist er hierfür mit einem seitlichen Fortsatz gebildet, der durch eine seitliche Durchbrechung des Stützkörpers 2 in den seitlichen Aufnahmeraum der Gehäusehüllstruktur 1 ragt, in dem der Motor 18, eine Steuerung und gegebenenfalls ein weitergehendes Gerätemanagementsystem untergebracht sind. Der im Wesentlichen hohlzylindrische Stützkörper 2 ist für diesen Zweck in seinem Mantel mit einer Durchbrechung versehen, durch die auch der Sensor 33 mit einer Sensoranschlussseite ragt, an der er mit der Steuerung und einer Anzeige verbunden ist.

Der Sensor 33 bildet einen elastischen Biegebalken, der an beiden Enden fest eingespannt ist. Der Halterung des Sensors 33 dient ein als einstückig geformter Sensorträger 37, der von dem Stützkörper 2 gleitend axial geführt wird und über eine Kontaktstelle 6 und den Sensor 33 gegen den rückwärtigen Öffnungsrand des Lagerkörpers 30 auf Anschlag oder gegebenenfalls mit dem Lagerkörper 30 fest verbunden ist. Da der Sensorträger 37 auch dann jede Axialbewegung des Lagerkörpers 30 mitmacht, wenn lediglich Anschlagkontakt besteht, wird er dem Lagerkörper 30 und damit der Fördereinrichtung zugerechnet.

Um das Axialspiel zwischen dem Stützkörper 2 und dem Lagerkörper 30 zu eliminieren oder zumindest auf ein Maß zu reduzieren, das in Bezug auf die Dosiergenauigkeit tolerierbar oder nach Möglichkeit in der Praxis nicht mehr spürbar ist, ist ein Kontaktelement 5 vorgesehen, das als weiteres Einstellglied 5 dient. Das Einstellglied 5 bildet die Kontaktstelle 6 für den Sensor 33. Die Kontaktstelle 6 ist ein Nocken, der auf der Translationsachse T von der Vorderseite des Einstellglieds 5 in Förderrichtung V vorragt. Der Lagerkörper 30 ist über den Sensor 33 axial lediglich an der Kontaktstelle 6 quasi punktuell auf der Translationsachse T abgestützt.

Das Einstellglied 5 ist in einem Einstelleingriff mit dem Stützkörper 2. Auch dieser Einstelleingriff ist im Ausführungsbeispiel ein Gewindeeingriff, nämlich zwischen einem Innengewinde 4 an dem rückwärtigen Ende des Stützkörpers 2 und einem entsprechenden Außengewinde des Einstellglieds 5. Das Einstellglied 5 ist eine Kreiszylinderscheibe, die axial gerade so dick ist, dass sie für einen ausreichend festen Einstelleingriff an ihrem äußeren Umfang mit einem ausreichend langen Gewindezug versehen ist.

Die Einstellposition des Einstellglieds 5 ist so gewählt, dass der Lagerkörper 30 gegen den Stützkörper 2 in Förderrichtung auf Anschlag ist und gleichzeitig die Kontaktstelle 6 die Rückseite des Kraftsensors 33 gerade berührt. Das Innengewinde 4 des Stützkörpers 2 ist ausreichend lang, um den Einstellkörper 5 einzuschrauben und im Einstelleingriff bis in diese Einstellposition einstellen zu können. Die Einstellposition ist vorzugsweise so gewählt, dass eine Eichkurve des kalibrierten Sensors 33 nicht verändert wird, insbesondere dass der Nullpunkt der Eichkurve erhalten bleibt. Der Offset des Sensors 33 ist mit anderen Worten daher "Null", wenn der Fluiddruck in dem Behältnis 12 dem Umgebungsdruck entspricht. Ein Offset wird erst bei einem Primen des Infusionsgeräts erhalten. Grundsätzlich kann die Einstellposition aber auch so gewählt sein, dass sich bereits in der Einstellposition ein Offset vor einem Primen einstellt. Dieser Einstelloffset sollte jedoch kleiner sein als der Offset, welcher sich bei einem Primen einstellt. Mit dem Begriff "Primen" wird der Vorgang bezeichnet, durch den die produktführenden Teile bis einschließlich zu einer Austrittsstelle des Katheters 8, der von einer Einstechkanüle oder einer weichen Kanüle gebildet werden kann, vollkommen mit dem Produkt gefüllt sind.

Das Einstellglied 5 ist in seiner Einstellposition an dem Stützkörper 2 festgelegt, vorzugsweise stoffschlüssig mit dem Stützkörper 2 verbunden. Der Stoffschluss kann beispielsweise durch Laserschweißung oder vorzugsweise durch ein in den Einstelleingriff eingebrachtes Adhäsionsmittel erhalten werden.

Auch ohne Okklusions- und/oder Leckerkennung ist ein Axialspiel nicht nur dem Gewindeeingriff zwischen dem Abtriebsglied 10 und dem Antriebsglied 20 inhärent, sondern auch den Drehlagerungen, wie sie von herkömmlichen Infusionsgeräten bekannt sind.

Um das Axialspiel in der Drehlagerung des Antriebsglieds 20 zu reduzieren, ist ein Einstellglied 35 vorgesehen. Das Einstellglied 35 ist in einem Einstelleingriff mit dem Lagerkörper 30. Auch dieser Einstelleingriff ist ein Gewindeeingriff. Wie bereits das Einstellglied 5 ist auch das Einstellglied 35 eine flache, scheibenförmige Schraube mit einem Außengewinde. Das Einstellglied 35 ist an dem rückwärtigen Ende des Lagerkörpers 30 in den Lagerkörper 30 eingeschraubt, der hierfür ein Innengewinde 34 bildet, das mit dem Außengewinde des Einstellkörpers 35 in dem Einstelleingriff ist. Das Einstellglied 35 ist so angeordnet, dass über die von der Drehlagerung ausgehenden Kräfte hinaus keine anderen äußeren Kräfte auf das Einstellglied 35 wirken können.

Für die axiale Abstützung und Festlegung des Antriebsglieds 20 bilden der Lagerkörper 30 eine gegen die Förderrichtung V weisende erste Stützfläche 31 und das Einstellglied 35 eine der Stützfläche 31 zugewandte zweite Stützfläche 32. Das Antriebsglied 20 bildet an seinem Steg 22 eine in die Förderrichtung V weisende dritte Stützfläche 23, die der ersten Stützfläche 31 zugewandt ist, und eine gegen die Förderrichtung V weisende vierte Stützfläche 24, die der zweiten Stützfläche 32 zugewandt ist. Zwischen den beiden Stützflächen 31 und 23 ist ein Kugellager 27 und zwischen den Stützflächen 32 und 24 ist ein weiteres Kugellager 28 axial gehalten. Jedes der Kugellager 27 und 28 bildet ein Radiallager und über die Stützflächen 31 und 23 sowie 32 und 24 ein Axiallager. Die Kugellager weisen wie üblich je einen inneren und einen äußeren Lagerring auf, die um die Translationsachse T relativ zueinander drehbar sind und zwischen denen je eine Mehrzahl von Kugeln angeordnet ist, die die Radial- und Axialkräfte zwischen den Lagerringen übertragen. Bei dem Kugellager 27 ist der innere Lagerring mit 27i und der äußere Lagerring mit 27a bezeichnet. Das Kugellager 28 weist entsprechend einen inneren Lagerring 28i und einen äußeren Lagerring 28a auf. Die inneren Lagerringe 27i und 28i sind radial an der äußeren Umfangsfläche des Abtriebsglieds 20 und die äußeren Lagerringe 27a und 28a sind radial an der gegenüberliegenden Mantelinnenfläche des Lagerkörpers 30 abgestützt.

Für die axiale Einspannung der Kugellager 27 und 28 ist das Einstellglied 35 in seiner Einstellposition mit einer geringen axialen Kraft gegen den äußeren Lagerring 28a gepresst. Das Einstellglied 35 und das Kugellager 28 sind nur mit dem äußeren Lagerring 28a und der zweiten Stützfläche 32 in Kontakt. Die zweite Stützfläche 32 ist eine geschlossen umlaufende, zu der Rotationsachse T konzentrische Ringstirnfläche eines Ringstegs 36, der in die Förderrichtung V von der Vorderseite des Einstellglieds 35 aufragt. Die Ringstirnfläche könnte auch Unterbrechungen aufweisen. Ebenso könnte die Stützfläche 32 durch einzeln aufragende Nocken gebildet sein.

Die äußeren Lagerringe 27a und 28a haben axial keinen Kontakt mit den Stützflächen 23 und 24 des Antriebsglieds 20. Die inneren Lagerringe 27i und 28i haben axial keinen Kontakt mit den Stützflächen 31 und 32. Der axiale Kraftfluss verläuft durch die Drehlagerung daher ausschließlich über den Kontakt der Stützflächen 31 und 32 mit dem jeweils zugewandten der äußeren Lagerringe 27a und 28a und den Kontakt zwischen den Stützflächen 23 und 24 und dem jeweils zugewandten der inneren Lagerringe 27i und 28i. Die axiale Kraft wird innerhalb der Kugellager 27 und 28 deshalb ausschließlich durch die Kugeln übertragen. Auf diese Weise entsteht abgesehen von Fertigungstoleranzen der Kugellager 27 und 28 eine axial nahezu spielfreie Drehlagerung.

Das Einstellglied 35 wird bereits wie die zuvor beschriebenen Einstellglieder in seiner Einstellposition gesichert. Die Sicherung an dem Lagerkörper 30 ist ebenfalls vorzugsweise stoffschlüssig und kann insbesondere durch ein Adhäsionsmittel bewirkt werden, das in den Einstelleingriff eingebracht ist. Andere Stoffschlussverbindungen, beispielsweise Laserschweißung, sind jedoch ebenfalls möglich. Wie bei den anderen Einstellgliedern wird die Sicherung im Einstelleingriff selbst vorgenommen.

Zu den Einstelleingriffen ist noch zu bemerken, dass die axialen Längen der Verstellwege der Einstellglieder 5,15, 25 und 35 in den Einstellgriffen je so lang sind, dass das jeweilige Einstellglied bei der Verstellung in die Einstellposition nicht gegen den Körper, mit dem es in dem Einstelleingriff ist, in einen Anschlagkontakt gelangen kann, der eine weitere Verstellung gleicher Richtung blockiert.

Noch eine weitere Quelle für ein der Dosiergenauigkeit abträgliches Axialspiel ist bei herkömmlichen Infusionsgeräten und auch bei herkömmlichen Injektionsgeräten der große Unterschied zwischen der axialen Wärmeausdehnung der Gehäuse und der axialen Wärmeausdehnung der eingesetzten Reservoirbehältnisse. Die Gehäuse sind üblicherweise aus Kunststoff im Spritzguss hergestellt, während die Behältnisse meist Glaskörper sind. Die Wärmeausdehnungskoeffizienten dieser Materialien unterscheiden sich im Allgemeinen etwa um den Faktor 10, d. h. um eine ganze Größenordnung. Für den axialen Ausgleich dieser Unterschiede in der Wärmeausdehnung sind die Behältnisse bei den herkömmlichen Geräten axial elastisch nachgiebig an den Gehäusen abgestützt. Im Einsatztemperaturbereich der Geräte, der immerhin den Bereich von -20°C bis 40°C abdeckt, ändern sich daher die Positionen zwischen den Fördereinrichtungen und den Behältnissen axial in einem Ausmaß, das die Dosiergenauigkeit spürbar beeinträchtigt.

Diesem Axialspiel und seiner für die Dosiergenauigkeit negativen Wirkung wird dadurch entgegengewirkt, dass der Stützkörper 2 in Axialrichtung eine Wärmeausdehnung aufweist, die deutlich näher bei der axialen Wärmeausdehnung des Behältnisses 12 liegt als die Gehäuse von herkömmlichen Geräten. So kann der Stützkörper 2 insbesondere aus einem Material bestehen, dessen Wärmeausdehnungskoeffizient sich höchstens um einen Faktor 5 von dem Wärmeausdehnungskoeffizient des Materials des Behältnisses 12 unterscheidet. Bevorzugter ist es, wenn die Wärmeausdehnungskoeffizienten so nah als möglich beieinander liegen oder gar gleich sind. Auch konstruktive Maßnahmen sind denkbar, beispielsweise die Fertigung des Stützkörpers 2 als Verbundkörper, der im Verbund mehrere Materialien aufweist, beispielsweise in Kunststoff eingelegte Aussteifungskörper, die die Wärmeausdehnung des Kunststoffmaterials in axialer Richtung behindern. Bevorzugte Materialien für den Erhalt einer günstigen Wärmeausdehnung weisen im Einsatztemperaturbereich einen Wärmeausdehnungskoeffizienten von 30 x 10⁻⁶ / K oder weniger auf. Die Materialien weisen vorzugsweise eine in alle Richtungen gleichmäßige Wärmeausdehnung auf. Eine Stützstruktur in Form eines Verbundkörpers wird allerdings naturgemäß eine ungleichmäßige Wärmeausdehnung, bezogen auf den gesamten Verbundkörper, aufweisen, so dass in solch einem Fall nur die axiale Wärmeausdehnung und der axiale Wärmeausdehnungskoeffizient gemeint sind.

Bevorzugte Konstruktionsmaterialien für Infusionsgeräte und Injektionsgeräte sind zusammen mit deren im Einsatztemperaturbereich geltenden Wärmeausdehnungskoeffizienten α in der folgenden Tabelle zusammengestellt:

| Material | Wärmeausdehnungskoeffizient α in 10⁻⁶ / K |
|---|---|
| Messing | 18 bis 19 |
| Stahl | 10 bis 12 |
| Aluminium | 23 bis 24 |
| Polyamid PA | 100 bis 140 |
| Polyoxymethylen POM | 110 bis 130 |
| Polyethylenterephthalat PET | 70 |
| Polycarbonat PC | 70 |
| Polytetrafluorethylen PTFE | 60 bis 200 |
| Acrylnitril/Butadien/Styrol ABS | 80 bis 110 |
| Glas | 5 bis 10 |
| Hartgummi | 75 bis 100 |

Durch einen Stützkörper 2 oder allgemeiner eine Stützstruktur 2 aus beispielsweise Aluminium oder einer Aluminium-Basislegierung kann bereits gegenüber denjenigen Kunststoffmaterialien, die in Bezug auf die Wärmeausdehnung dem Behältnismaterial, vorzugsweise Glas, am nächsten kommen, eine deutliche Verbesserung erzielt werden, da der Wärmeausdehnungskoeffizient von Aluminium um etwa den Faktor 3 kleiner ist als der Wärmeausdehnungskoeffizient der den Behältnismaterial in Bezug auf den Wärmeausdehnungskoeffizient am nächsten kommenden Kunststoffmaterialien. Durch die Verwendung eines Messingmaterials ist eine weitere Verbesserung erzielbar. Wird die Stützstruktur aus Stahl gebildet oder weist sie Stahlkomponenten in solch einer Anordnung auf, dass durch die Stahlkomponenten die axiale Wärmeausdehnung maßgeblich beeinflusst wird, so kann bei entsprechender Wahl des Glasmaterials im günstigsten Fall sogar eine identische Wärmeausdehnung erzielt werden. Wird der Stützkörper 2 oder allgemeiner eine Stützstruktur 2, die natürlich ebenfalls axiale Stützfunktion wie der Stützkörper 2 übernimmt, als Verbundkörper gebildet, so kann durch Aussteifungskörper, beispielsweise in eine Kunststoffmatrix eingelagerte Axialfasern, ein vergleichbar günstiges Wärmeausdehnungsverhalten erzielt werden, wenn der oder die Aussteifungskörper eine Wärmeausdehnung wie vorstehend beschrieben aufweisen.

Auf die Mehrteiligkeit des Gehäuses, im Ausführungsbeispiel Zweiteiligkeit, kann grundsätzlich sogar verzichtet werden, wenn die Gehäusehülle, im Ausführungsbeispiel die Hüllstruktur 1, eine erfindungsgemäße Wärmeausdehnung besitzt. Bei solch einer Ausbildung einer Gehäusehülle wird es bevorzugt, wenn die Gehäusehülle als Verbundkörper gebildet ist, beispielsweise als Kunststoffinatrix mit eingelegten Aussteifungskörpern wie insbesondere axial orientierte Metallfasern.

Wenngleich bereits allein eine Stützstruktur vorteilhaft ist, die nur das Behältnis axial abstützt, ist es vorteilhafter, wenn solch eine Stützstruktur sich über eine möglichst große, in Förderrichtung V des Kolbens 13 gemessene Länge erstreckt. Die Stützstruktur sollte, wie beispielhaft der Stützkörper 2, zusätzlich auch noch die Fördereinrichtung axial in beide Richtungen abstützen. Besonders günstig ist es ferner, wenn auch die Fördereinrichtung im Ganzen eine axiale Wärmeausdehnung aufweist, die möglichst nah bei der axialen Wärmeausdehnung der Stützstruktur angesiedelt ist, beispielsweise indem die Stützstruktur und die Komponenten der Fördereinrichtung aus dem gleichen Material oder aus gegebenenfalls unterschiedlichen Materialien mit möglichst nahe beieinander liegender axialer Wärmeausdehnung gefertigt sind. Vorteilhafterweise weist das Abtriebsglied 10 oder das Antriebsglied 20 oder weisen vorzugsweise diese beiden Komponenten je eine im wesentlichen gleiche axiale Wärmeausdehnung wie der Stützkörper 2 auf, d.h. eine Wärmeausdehnung, die sich um höchstens den Faktor 5 und bevorzugter um weniger als den Faktor 5, möglichst um höchstens den Faktor 2 oder noch weniger von der axialen Wärmeausdehnung des Stützkörpers 2 unterscheidet und idealerweise gleich ist.

Je größer die von der bevorzugt einteiligen Stützstruktur oder den mehreren Stützkörpern einer mehrteiligen Stützstruktur erst gemeinsam überbrückte axiale Länge ist, desto geringer ist das auf unterschiedliche axiale Wärmeausdehnungen zurückzufiihrende Axialspiel. Kunststoffteile herkömmlicher Art dürfen hierbei durchaus kurze axiale Längen überbrücken. Je kürzer die von herkömmlichen Kunststoffteilen überbrückten axialen Längen sind, desto geringer ist das auf unterschiedliche Wärmeausdehnungen zurückzuführende Axialspiel. Besonders günstig ist es, wie im Ausführungsbeispiel, wenn solch ein Stützkörper oder gegebenenfalls auch mehrere axial hintereinander angeordnete Stützkörper vorgesehen ist oder sind, der oder die bezüglich ihrer axialen Wärmeausdehnung nahe bei derjenigen des Behältnisses und/oder der Fördereinrichtung liegen. Die Stützeinrichtungen des Stützkörpers, die das Behältnis und/oder die Fördereinrichtung axial an dem Stützkörper oder den Stützkörpern festlegen, sollten von jeweiligen dem Stützkörper in einem Stück gebildet oder mit dem jeweiligen Stützkörper so verbunden sein, dass sie relativ zu dem Stützkörper axial nicht bewegbar sind, wie beispielsweise das Paar der Stege 3 und 7a, das Paar aus Stützsteg 3 und Einstellglied 5 und das Paar aus Einstellglied 35 und Stützfläche 31.

Der Stützkörper 2 ist ein vergleichsweise einfacher Hülsenkörper, der in die Hüllstruktur 1 eingesetzt ist und lediglich der Lagerung der relativ zueinander axial bewegbaren Teile und damit zur axialen Aussteifung vorgesehen ist. Die Hüllstruktur 1 selbst kann wie üblich aus Kunststoff im Spritzguss hergestellt sein. Die Hüllstruktur 1 besteht aus zwei Teilen, nämlich einem Hauptteil und einem Bodenteil. Das Hauptteil bildet die Aufnahmekammer für die Stützstruktur 2 und die gegebenenfalls nicht von der Stützstruktur 2 gelagerten Komponenten der Verabreichungsvorrichtung. Das Bodenteil ist eine einfache Platte, die mit dem Hauptteil an dessen Rückseite fest verbunden ist und dort die Aufnahmekammer verschließt.

Der Deckel 7 besteht vorzugsweise aus dem gleichen Material wie der Stützkörper 2. Ebenso gilt dies für den Lagerkörper 30, die beiden Einstellglieder 35 und 5 und die Trägerscheibe 37, so dass eine insgesamt in Bezug auf die axiale Wärmesausdehnung sehr homogene Stützstruktur erhalten wird. Eine Fertigung des Deckels 7 und/oder der Trägerscheibe 37 und/oder des Einstellglieds 35 und/oder des Einstellglieds 5 aus einem der üblichen Kunststoffmaterialien ist jedoch weit weniger kritisch als die Bildung einer axial langen Stützstruktur aus den herkömmlichen Kunststoffmaterialien.

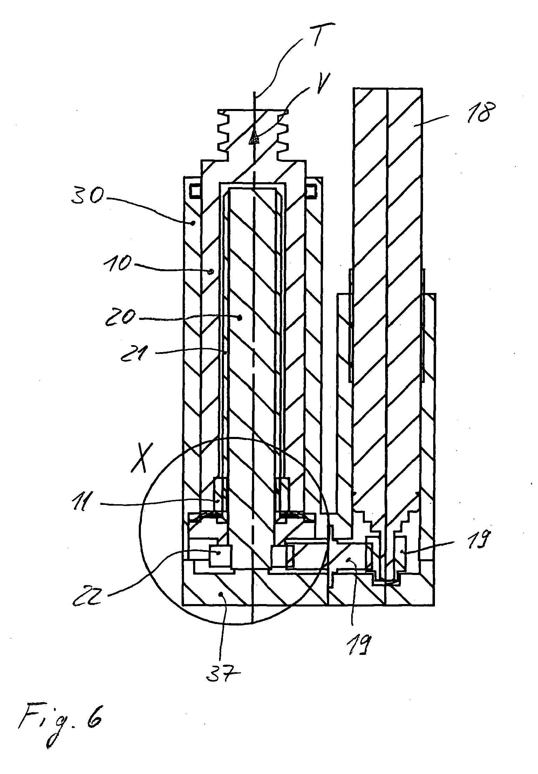

Figur 6 zeigt in einem Längsschnitt einen wie im ersten Ausführungsbeispiel gelagerten Lagerkörper 30 mit den von ihm gelagerten Komponenten einer Verabreichungsvorrichtung nach einem zweiten Ausführungsbeispiel, die ebenfalls ein Infusionsgerät ist. Diejenigen Komponenten des zweiten Ausführungsbeispiels, die ihrer Funktion und zum Teil auch Konstruktion nach mit Komponenten des ersten Ausführungsbeispiels vergleichbar sind, tragen die gleichen Bezugszeichen wie im ersten Ausführungsbeispiel. Unterschiede bestehen nur soweit nachfolgend hierauf aufmerksam gemacht wird oder aus den Figuren selbst sich ergibt. Es sollen die Ausführungen zum ersten Ausführungsbeispiel gelten, soweit sich hieraus keine offensichtlichen Widersprüche ergeben.

Die Verabreichungsvorrichtung des zweiten Ausführungsbeispiels weist eine Spielreduziereinrichtung lediglich für die Eliminierung oder zumindest Reduzierung des Axialspiels zwischen dem Rotationsglied 20 und dem Lagerkörper 30 auf. Die Spielreduziereinrichtung spannt das Rotationsglied 20 im Unterschied zum ersten Ausführungsbeispiel axial unmittelbar gegen den Sensorträger 37. Ferner umgibt in dem zweiten Ausführungsbeispiel das Translationsglied 10 das Rotationsglied 20. Das Translationsglied 10 und das Rotationsglied 20 sind miteinander in einem Gewindeeingriff. Hierfür ist das Rotationsglied 20 über den größten Teil seiner axialen Länge mit einem Außengewinde 21 und das Translationsglied 10 nur an seinem bezüglich der Translationsrichtung V rückwärtigen Ende mit einem Innengewinde 11 versehen. Das Translationsglied 10 ist an dem Lagerkörper 30 axial linear geführt. Wie bei dem ersten Ausführungsbeispiel treibt ein Motor 18, vorzugsweise ein elektrischer Schrittmotor, über ein Stirnradgetriebe mit zwei in Stirneingriff befindlichen Zahnrädern 19 das Rotationsglied 20 um die Rotations- und Translationsachse T rotatorisch an. Das Rotationsglied 20 ist für seinen Antrieb an seinem rückwärtigen Ende wieder mit einem umlaufenden außenverzahnten Ringsteg 22 versehen, der mit dem Zwischenrad 19 des Stirnradgetriebes für den rotatorischen Antrieb des Rotationsglieds 20 in einem Stirneingriff ist.

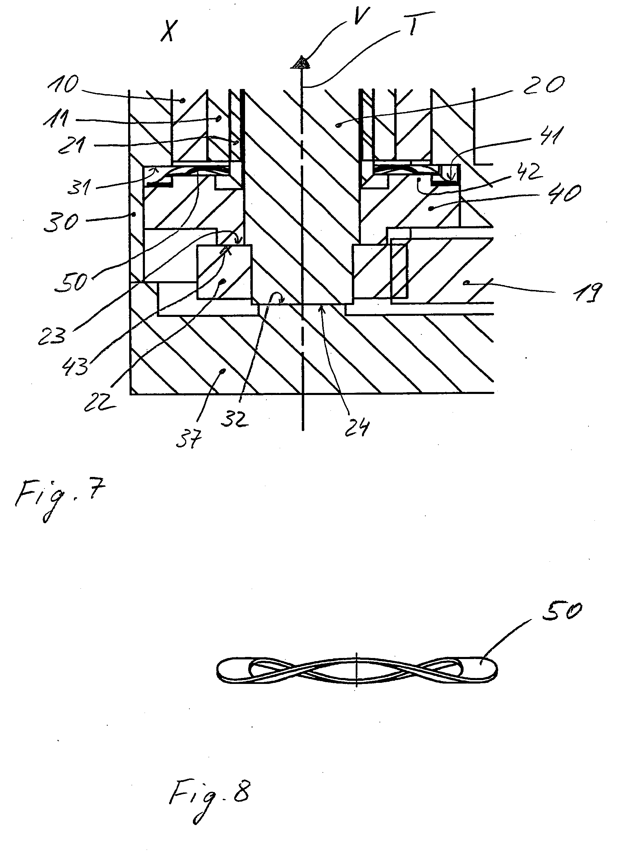

Die Drehlagerung des Rotationsglieds 20 ist in Figur 7 vergrößert dargestellt. Die Drehlagerung des zweiten Ausführungsbeispiels ist als einfache Gleitlagerung gebildet. Die zweite Stützfläche 32 des Sensorträgers 37 und die vierte Stützfläche 24 des Rotationsglieds 20 bilden ein erstes Gleitpaar der Drehlagerung. Die beiden Stützflächen 32 und 24 befinden sich unmittelbar miteinander in Gleitkontakt. Die zweite Stützfläche 32 ist an dem rückwärtigen Stirnende des Rotationsglieds 20 gebildet. Von dem Sensorträger 37 ragt ihr entgegen ein kurzer Sockel auf, dessen Stirnfläche die zweite Stützfläche 24 bildet. Der Sockel stellt das Rotationsglied 20 von dem Sensorträger 37 frei. Die Formung eines Sockels ermöglicht eine präzisere Fertigung der zweiten Stützfläche 32. Die dritte Stützfläche 23 ist in der Art wie die Stützfläche 23 des ersten Ausführungsbeispiels gebildet, nämlich durch die in Translationsrichtung T weisende vordere Stirnfläche des Ringstegs 22. Die ihr axial zugewandte erste Stützfläche 31 wird von dem Lagerkörper 30 gebildet. Allerdings sind die Stützflächen 31 und 23 radial zueinander versetzt, d.h. sie liegen axial nicht exakt in der Flucht. Der Radialversatz wird von einem Übertragungskörper 40, im Ausführungsbeispiel ein Übertragungsring, überbrückt, der zwischen den Stützflächen 31 und 23 angeordnet ist. Der Übertragungskörper 40 bildet an einer vorderen Stirnseite der ersten Stützfläche 31 axial in der Flucht gegenüberliegend eine um die Translationsachse T und das Rotationsglied 20 umlaufende vordere Stützfläche 41 und an seiner Rückseite der dritten Stützfläche 23 axial in der Flucht gegenüberliegend eine rückwärtige Stützfläche 43. Die rückwärtige Stützfläche 43 ist unmittelbar auf Anschlagkontakt mit der dritten Stützfläche 23. Zwischen der ersten Stützfläche 31 und der ihr zugewandten vorderen Stützfläche 41 verbleibt ein lichter, axialer Abstand. Zwischen den beiden Stützflächen 31 und 41 ist eine Ringfeder 50 angeordnet, die sich axial an beiden Stützflächen 31 und 41 mit einer axialen Vorspannkraft abstützt.

Die Ringfeder 50 ist in Figur 8 einzeln dargestellt. Sie ist umlaufend wellenförmig und beispielsweise aus Federstahl gefertigt. Im eingebauten Zustand stützt sie sich mit ihren Wellenbergen und Wellentälern umlaufend alternierend an der ersten Stützfläche 31 und der vorderen Stützfläche 41 des Übertragungskörpers 40 ab. Bei axialer Stauchung wirkt sie wie eine Blattfeder.

Wie insbesondere in Figur 7 erkennbar, dient der Übertragungskörper 40 nicht nur dem Ausgleich des Radialversatzes, sondern auch der Zentrierung der Ringfeder 40. Hierfür ist der Übertragungskörper 40 an seiner vorderen Stirnseite mit einem umlaufenden Vorsprung 42 versehen, um dessen äußeren Umfang die vordere Stützfläche 41 axial ein Stück weit zurückgenommen umläuft.

Die Ringfeder 50 und der Übertragungskörper 40 bilden die Spielreduziereinrichtung des zweiten Ausführungsbeispiels, indem der Übertragungskörper 40 relativ zu dem Lagerkörper 30 axial bewegbar ist. Vorzugsweise wird er von dem Lagerkörper 30 axial linear geführt. Grundsätzlich kann er jedoch relativ zu dem Lagerkörper 30 drehbeweglich sein. Obgleich der Übertragungskörper 40 grundsätzlich mit dem Rotationsglied 20 drehsteif verbunden sein kann, entweder mittels Fügeverbindung oder durch eine einstückige Ausbildung mit dem Rotationsglied 20, wird es bevorzugt, wenn der Übertragungskörper 40 wie im Ausführungsbeispiel relativ zu dem Rotationsglied 20 drehbeweglich und noch bevorzugter auch axial beweglich angeordnet ist. Auf diese Weise wird ein weiteres Gleitflächenpaar der Drehlagerung durch die unmittelbar aneinander abgleitenden Stützflächen 23 und 43 gebildet. Die Ringfeder 50 wird somit vorteilhafterweise von Drehbewegungen freigehalten.

Im zweiten Ausführungsbeispiel wird im Ergebnis eine vorteilhaft einfache Spielreduziereinrichtung für das Rotationsglied 20 erhalten, die bei ausreichender Vorspannung der Ringfeder 50 jegliches Axialspiel zwischen dem Rotationsglied 20 und dem Lagerkörper 30 eliminiert. In Ausführungen, in denen die Stützflächen 31 und 23 axial einander in der Flucht gegenüberliegen, könnte auf den Übertragungskörper 40 verzichtet werden. Um die Ringfeder 50 jedoch auch in solchen Ausführungen von Drehbewegungen freizuhalten und/oder eine einfach fertigbare Zentrierung für die Ringfeder 50 oder auch eine die Presskraft erzeugende andere Federeinrichtung zu erhalten, ist die Zwischenschaltung eines Übertragungskörpers in der Art des Übertragungskörpers 40 auch dann von Vorteil.

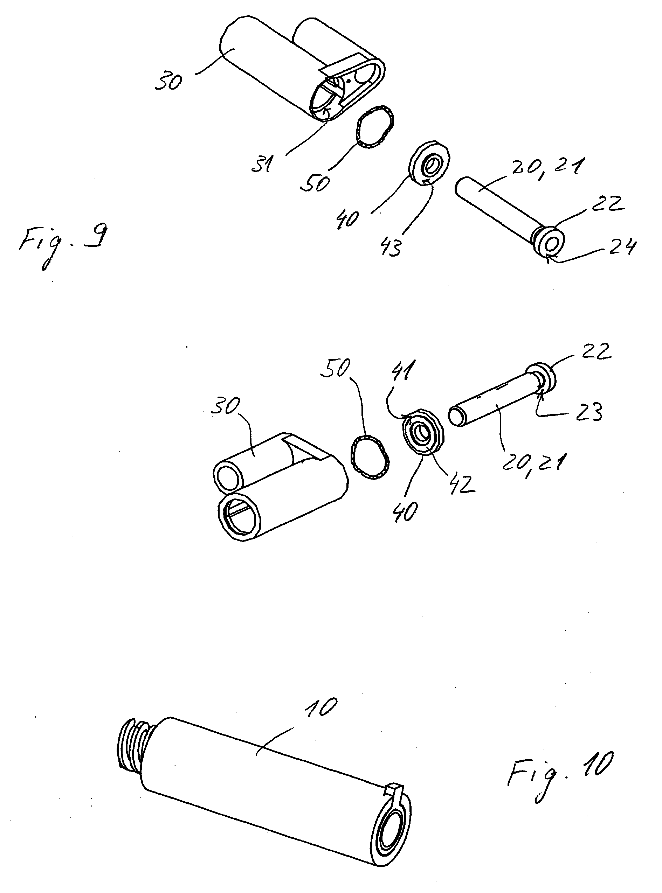

Figur 9 zeigt in einer Explosionsdarstellung den Lagerkörper 30, das Rotationsglied 20, den Übertragungskörper 40 und die Ringfeder 50 entlang der zu denkenden Translationsachse in einer für die Montage geeigneten Folge aufgereiht. Figur 10 zeigt das Translationsglied 10 einzeln. Bei der Montage kann in einem ersten Schritt das Translationsglied 10 alleine von hinten in den Lagerkörper eingesetzt und anschließend das Rotationsglied 20 in das Translationsglied 10 eingeschraubt werden, oder es kann zuerst die Gewindeverbindung zwischen dem Translationsglied 10 und dem Rotationsglied 20 hergestellt und erst anschließend das Translationsglied 10 mit dem eingeschraubten Rotationsglied 20 in den Lagerkörper 30 eingesetzt werden. Vor dem Einschrauben des Rotationsglieds 20 werden der Übertragungskörper 40 und die Ringfeder 50 über das Außengewinde 21 bis gegen den Ringsteg 22 des Rotationsglieds 20 geschoben, und anschließend wird das Rotationsglied 20 in das Translationsglied 10 eingeschraubt. Nachdem das Translationsglied 10 und das Rotationsglied 20 in dem Lagerkörper 30 angeordnet sind, wird der Sensorträger 37 mit dem in Figur 9 gezeigten Hauptteil des Lagerkörpers 30 verbunden, so dass es die zum Zwecke der Montage offene Rückseite des Lagerkörpers 30 verschließt. Lagerkörper 30 und Sensorträger 37 sind im verbundenen Zustand relativ zueinander nicht bewegbar. Die Verbindung ist ferner so gestaltet, dass die Ringfeder 50 mit einer definierten axialen Vorspannkraft eingebaut ist.

Der Gewindeeingriff der Gewinde 11 und 21 ist im zweiten Ausführungsbeispiel als einfacher Gewindeeingriff gebildet, kann jedoch ohne weiteres wie bei dem ersten Ausführungsbeispiel mittels einer zusätzlich Spielreduziereinrichtung axialspielreduzierend gebildet sein.

Schließlich sei auch darauf hingewiesen, dass bei dem ersten Ausführungsbeispiel eine Spielreduziereinrichtung für die Reduzierung oder vorzugsweise Eliminierung von Axialspiel der Drehlagerung des Rotationsglieds 20 wie bei dem zweiten Ausführungsbeispiel gebildet sein kann und dass umgekehrt die auf dem verstellbaren Einstelleingriff beruhende Spielreduziereinrichtung 35 beim zweiten Ausführungsbeispiel statt der Spielreduziereinrichtung 40, 50 vorgesehen sein kann. Auch Mischformen sind denkbar. So könnte eines der Wälzlager 27 und 28 zwischen einem der Stützflächenpaare 31, 23 und 32, 24 oder je solch ein Wälzlager zwischen beiden Stützflächenpaaren angeordnet sein, wobei das oder die beiden Wälzlager vorzugsweise je wie die Wälzlager 27 und 28 des ersten Ausführungsbeispiels angeordnet wären, d.h. es würde die Ringfeder 50 oder eine alternative Feder nur auf eine der Lagerschalen solch eines Wälzlagers wirken.

Der Lagerkörper 30 kann unter Verzicht auf die axial bewegliche Lagerung und den Sensor 33 zu einem Gehäuse mit einer Aufnahme für ein Reservoir 12 abgewandelt werden und dann direkt als Hüllstruktur wie die Hüllstruktur des ersten Ausführungsbeispiels dienen. Solch eine Hüllstruktur kann wie herkömmliche Gehäuse gebildet sein. Alternativ kann sie jedoch die Wärmeausdehnungseigenschaften der Stützstruktur 2 des ersten Ausführungsbeispiels haben, so dass auf die diesbezüglichen Ausführungen zum ersten Ausführungsbeispiel verwiesen wird.

### Bezugszeichen:

- 1: Gehäusestruktur, Hüllstruktur
- 2: Gehäusestruktur, Stützstruktur, Stützkörper
- 3: Stützschulter, Stützsteg
- 4: Gewinde
- 5: Einstellglied, Kontaktelement
- 6: Kontaktstelle
- 7: Abschlusselement, Deckel
- 7a: Gegenstützschulter, Gegenstützsteg
- 8: Katheter
- 9: Eingriffseinrichtung, Gewinde
- 10: Abtriebsglied, Translationsglied
- 11: Gewinde
- 11f: Eingriffsflanke
- 12: Reservoir, Behältnis
- 13: Kolben
- 14: Auslass
- 15: Einstellglied
- 16: Eingriffsglied
- 17: Rückstellelement
- 18: Motor
- 19: Zahnräder
- 20: Antriebsglied, Rotationsglied
- 21: Gewinde
- 21f: Eingriffsflanke
- 22: Steg
- 23: Stützfläche
- 24: Stützfläche
- 25: Einstellglied
- 25f: Eingriffsflanke
- 26: Gewinde
- 27: Wälzlager
- 27a: äußerer Lagerring
- 27i: innerer Lagerring
- 28: Wälzlager
- 28a: äußerer Lagerring
- 28i: innerer Lagerring
- 29: Führungsbahn
- 30: Lagerkörper
- 31: Stützfläche
- 32: Stützfläche
- 33: Sensor
- 34: Gewinde
- 35: Einstellglied
- 36: Steg
- 37: Sensorträger
- 38: -
- 39: -
- 40: Übertragungskörper
- 41: vordere Stützfläche
- 42: Vorsprung
- 43: rückwärtige Stützfläche
- 44-49: -
- 50: Feder

- T: Translationsachse, Rotationsachse
- V: Förderrichtung

## Patentansprüche

1. Vorrichtung für die dosierte Verabreichung eines fluiden Produkts, die Vorrichtung umfassend:
a) ein Behältnis (12) für das Produkt,
b) eine Fördereinrichtung (10, 13, 20), die auf das in dem Behältnis (12) befindliche Produkt wirkt und mittels einer axialen Abtriebsbewegung in eine Förderrichtung (V) Produkt aus dem Behältnis (12) fördert,
c) und eine in sich axial steife Gehäusestützstruktur (2), die das Behältnis (12) in und gegen die Förderrichtung (V) abstützt,
d) wobei die Gehäusestützstruktur (2) über die axiale Länge des Behältnisses (12) eine axiale Wärmeausdehnung aufweist, die in einem Einsatztemperaturbereich der Vorrichtung zumindest im Wesentlichen der axialen Wärmeausdehnung des Behältnisses (12) entspricht.

2. Vorrichtung nach Anspruch 1, **dadurch gekennzeichnet, dass** die axiale Wärmeausdehnung der Gehäusestützstruktur (2) und die axiale Wärmeausdehnung des Behältnisses (12) sich um höchstens 500% voneinander unterscheiden.

3. Vorrichtung nach einem der vorhergehenden Ansprüche, **dadurch gekennzeichnet, dass** die Gehäusestützstruktur (2) aus einem Material besteht, das in dem Einsatztemperaturbereich einen Wärmeausdehnungskoeffizienten hat, der zumindest im Wesentlichen dem Wärmeausdehnungskoeffizienten des Materials entspricht, aus dem das Behältnis (12) besteht.

4. Vorrichtung nach dem vorhergehenden Anspruch, **dadurch gekennzeichnet, dass** der Wärmeausdehnungskoeffizient des Materials der Gehäusestützstruktur (2) und der Wärmeausdehnungskoeffizient des Materials des Behältnisses (2) sich um höchstens 500% voneinander unterscheiden.

5. Vorrichtung nach einem der vorhergehenden Ansprüche, **dadurch gekennzeichnet, dass** eine quer zu der Förderrichtung (V) weisende, als Anschlag für das Behältnis (12) dienende Stützschulter (3) von der Gehäusestützstruktur (2) in einem Stück gebildet wird oder mit der Gehäusestützstruktur in einem lösbaren, axial steifen Eingriff ist.

6. Vorrichtung nach dem vorhergehenden Anspruch, **dadurch gekennzeichnet, dass** eine quer zu der Förderrichtung (V) weisende, für das Behältnis (12) als Gegenanschlag zu der Stützschulter (3) dienende Stützgegenschulter (7a) in einem lösbaren, axial steifen Eingriff mit der Gehäusestützstruktur (2) ist oder von der Gehäusestützstruktur in einem Stück gebildet wird.

7. Vorrichtung nach einer Kombination der zwei vorhergehenden Ansprüche, **dadurch gekennzeichnet, dass** die Stützschulter (3) und die Stützgegenschulter (7a) das Behältnis (12) in und gegen die Förderrichtung (V) fixieren.

8. Vorrichtung nach einem der vorhergehenden Ansprüche, **dadurch gekennzeichnet, dass** die Gehäusestützstruktur (2) ein Aufnahmefach mit einer quer zu der Förderrichtung (V) einwärts ragenden Stützschulter (3) und einer der Stützschulter (3) gegenüberliegenden Öffnung bildet, durch die das Behältnis (12) in das Aufnahmefach eingesetzt werden kann, und dass die Vorrichtung ein Abschlusselement (7) aufweist, das in einem lösbaren Eingriff mit einer Eingriffseinrichtung (9) der Gehäusestützstruktur (2) die Öffnung des Aufnahmefachs abschließt und eine der Stützschulter (3) gegenüberliegende Stützgegenschulter (7a) für das Behältnis (12) bildet.

9. Vorrichtung nach einem der drei vorhergehenden Ansprüche, **dadurch gekennzeichnet, dass** die Gehäusestützstruktur (2) in einem Axialabschnitt, der sich zumindest von der Stützschulter (3) bis zu der Stützgegenschulter (7a) erstreckt, die zumindest im Wesentlichen gleiche Wärmeausdehnung aufweist.

10. Vorrichtung nach einem der vier vorhergehenden Ansprüche, **dadurch gekennzeichnet, dass** die Gehäusestützstruktur (2) in einem Axialabschnitt, der sich zumindest von der Stützschulter (3) bis zu der Stützgegenschulter (7a) erstreckt, in einem Stück gebildet ist.

11. Vorrichtung nach einem der vorhergehenden Ansprüche, **dadurch gekennzeichnet, dass** die Gehäusestützstruktur (2) die Fördereinrichtung (10, 13, 20) axial abstützt.

12. Vorrichtung nach dem vorhergehenden Anspruch, **dadurch gekennzeichnet, dass** die Vorrichtung einen Kraftsensor (33) für eine Okklusions- und/oder Leckdetektion umfasst, und dass die Fördereinrichtung (10, 13, 20) über den Kraftsensor (33) gegen die Förderrichtung (V) an der Gehäusestützstruktur (2) abgestützt ist.

13. Vorrichtung nach dem vorhergehenden Anspruch, **dadurch gekennzeichnet, dass** die Fördereinrichtung (10, 13, 20) gegen eine elastische Rückstellkraft des Kraftsensors (33) relativ zu der Gehäusestützstruktur (2) gegen die Förderrichtung (V) bewegbar ist.

14. Vorrichtung nach einem der zwei vorhergehenden Ansprüche, **dadurch gekennzeichnet, dass** die Vorrichtung ein Kontaktelement (5) umfasst, gegen das der Kraftsensor (33) drückt, wenn die Fördereinrichtung (10, 13, 20) die Abtriebsbewegung ausführt, und dass das Kontaktelement (5) oder der Kraftsensor mit der Gehäusestützstruktur (2) oder der Fördereinrichtung in einem Einstelleingriff in solch eine Einstellposition verstellt und in der Einstellposition so gesichert ist, dass ein Axialspiel zwischen der Fördereinrichtung (10, 13, 20) und der Gehäusestützstruktur (2) verringert ist.

15. Vorrichtung nach einem der vier vorhergehenden Ansprüche, **dadurch gekennzeichnet, dass** die Gehäusestützstruktur (2) die Fördereinrichtung (10, 13, 20) axial führt.

16. Vorrichtung nach einem der fünf vorhergehenden Ansprüche, **dadurch gekennzeichnet, dass** die Gehäusestützstruktur (2) in die Förderrichtung (V) einen Anschlag für die Fördereinrichtung (10, 13, 20) bildet.

17. Vorrichtung nach einem der sechs vorhergehenden Ansprüche, **dadurch gekennzeichnet, dass** die Gehäusestützstruktur (2) zumindest in einem Axialabschnitt, der die Fördereinrichtung (10, 13, 20) in und gegen die Förderrichtung (V) abstützt, in einem Stück gebildet ist.

18. Vorrichtung nach einem der vorhergehenden Ansprüche, **dadurch gekennzeichnet, dass** die Vorrichtung einen Lagerkörper (30) umfasst, der die Fördereinrichtung (10, 13, 20) axial abstützt und den die Gehäusestützstruktur (2) axial führt.

19. Vorrichtung nach dem vorhergehenden Anspruch, **dadurch gekennzeichnet, dass** die Gehäusestützstruktur (2) zumindest über einen Axialabschnitt, der den Lagerkörper (30) in und gegen die Förderrichtung (V) abstützt, eine axiale Wärmeausdehnung aufweist, die zumindest im Wesentlichen der axialen Wärmeausdehnung des Lagerkörpers (30) über dessen zwischen den Orten der Abstützung gemessenen Axialabschnitt entspricht.

20. Vorrichtung nach dem vorhergehenden Anspruch, **dadurch gekennzeichnet, dass** der Lagerkörper (30) in seinem Axialabschnitt pro axialer Längeneinheit eine axiale Wärmeausdehnung aufweist, die zumindest im wesentlichen der pro axialer Längeneinheit gemessenen axialen Wärmeausdehnung des Behältnisses (12) entspricht.

21. Vorrichtung nach einem der vorhergehenden Ansprüche, **dadurch gekennzeichnet, dass** die Fördereinrichtung ein Antriebsglied (20) und ein axial in Förderrichtung (V) in das Behältnis (12) ragendes Abtriebsglied (10) umfasst, die miteinander in solch einem Eingriff sind, dass eine Antriebsbewegung des Antriebsglieds (20) die von dem Abtriebsglied (10) ausgeführte Abtriebsbewegung bewirkt, und dass die Stützstruktur (2) zumindest über einen Axialabschnitt, der sich von dem Eingriff zwischen dem Antriebsglied (20) und dem Abtriebsglied (10) bis zu einem in Förderrichtung (V) vorderen Ende des Abtriebsglieds (10) erstreckt, eine axiale Wärmeausdehnung aufweist, die zumindest im Wesentlichen der zwischen dem Eingriff und dem vorderen Ende des Abtriebsglieds (10) gemessenen axialen Wärmeausdehnung des Abtriebsglieds (10) entspricht.

22. Vorrichtung nach dem vorhergehenden Anspruch, **dadurch gekennzeichnet, dass** die Stützstruktur (2) in einem Axialabschnitt, der sich von einer Lagerstelle des Antriebsglieds (20) bis zu dem vorderen Ende des Abtriebsglieds (10) erstreckt, zumindest im Wesentlichen die gleiche Wärmeausdehnung hat wie die Fördereinrichtung, gemessen von der Lagerstelle des Antriebsglieds (20) bis zu dem vorderen Ende des Abtriebsglieds (10).

23. Vorrichtung nach einem der zwei vorhergehenden Ansprüche, **dadurch gekennzeichnet, dass** das Abtriebsglied (10) von dem Eingriff bis zu seinem vorderen Ende pro axialer Längeneinheit eine axiale Wärmeausdehnung aufweist, die zumindest im Wesentlichen der pro axialer Längeneinheit gemessenen axialen Wärmeausdehnung des Behältnisses (12) entspricht.

24. Vorrichtung nach einem der vier vorhergehenden Ansprüche, **dadurch gekennzeichnet, dass** die miteinander verglichenen axialen Wärmeausdehnungen sich um höchstens 500%, vorzugsweise höchstens 300%, unterscheiden.

25. Vorrichtung nach einem der vorhergehenden Ansprüche, **dadurch gekennzeichnet, dass** das eine Gehäusehüllstruktur (1) die Gehäusestützstruktur (2) umgibt.
